# EUROPEAN PATENT APPLICATION

(11) **EP 1 547 581 A1**
(43) Date of publication of application: **29.06.2005**
(21) Application number: 03029802.0
(22) Date of filing: 23.12.2003
(51) Int. Cl.: A61K 9/127

(54) **Liposomal vaccine for the treatment of human hematological malignancies**

(71) Applicant: Vectron Therapeutics AG, 35037 Marburg (DE)
(72) Inventor: Müller, Rolf, Prof., 35037 Marburg (DE); Graser, Andreas, 79618 Rheinfelden (Baden) (DE); Konur, Abdo, Dr., 35037 Marburg (DE); Müller-Brüsselbach, Sabine, Dr., 35037 Marburg (DE)
(74) Representative: Zwicker, Jörk

(57) **Abstract**

The present invention relates to liposomes and compositions comprising liposomes, their production and use for the prevention and therapy of proliferative diseases, infectious diseases, vascular diseases, rheumatoid diseases, inflammatory diseases, immune diseases, and allergies.

## Description

### Background of the Invention

### 1. Field of the Invention

The present invention relates to liposomes and compositions comprising liposomes, their production and use for the prevention and therapy of proliferative diseases, infectious diseases, vascular diseases, rheumatoid diseases, inflammatory diseases, immune diseases, and allergies. The liposomes and compositions are particularly useful for the delivery of therapeutic agents, preferably antigens to cells of the hematopoietic system.

### 2. Description of Related Art

Currently most therapeutic agents are administered to the patient in a free form which means that they are in solution and not attached or incorporated into a vehicle. The term "free form" also comprises chemical derivatives of a given therapeutic agent as well as various addition salts that can be formed with the therapeutic agent. However, it has been realized that the attachment of a therapeutic agent to or the incorporation of a therapeutic agent into a delivery vehicle can offer advantages if compared to the administration of the therapeutic agent, in particular and antigen in its free form. Several factors, which influence the overall efficacy of a given therapeutic agent, can be advantageously affected by incorporation into or attachment to such a vehicle. These factors include tissue specific distribution, in particular preferential accumulation in a certain tissue of interest or at a disease site, targeting of the drug or antigen to a particular cell type, and in particular for the delivery of antigens uptake by antigen presenting hemopoietic cells, like for example, Dendritic cells (DC) or Langerhans cells (LC). When comparing the suitability of different delivery vehicles the above factors are important but among such delivery vehicles also factors such as the release characteristic of the therapeutic agent from the vehicle become important for their efficacy.

While all of these factors will contribute to some degree to a potential improvement of the efficacy of a given therapeutic agent, when attached to or incorporated into such a delivery vehicle, the ultimate test for a novel delivery vehicle is its efficacy in a disease animal model or in a patient when compared to the therapeutic agent in its "free form" or to another vehicle.

One type of delivery vehicle, which has gained attention in recent years are liposomes and liposomal formulations. The term "liposomes" generally refers to uni- or multilamellar lipid structures enclosing an aqueous interior, depending on the number of lipid membranes formed. Typically liposomes can be loaded with therapeutic agents, i.e. the therapeutic agent is encapsulated in the interior of the liposome, and/or therapeutic agents can be attached to the liposome or incorporated into the lipid bilayer. Such liposomal formulations have been shown to have an increased efficacy in comparison to the free drug. For example, it has been shown that a liposomal formulation including the vinca alkaloid vincristine has a greater efficacy against leukemia cells, if compared to free vincristine and that it shows a reduced overall toxicity (Mayer *et al*. (1993) Cancer Chemo. Pharmacol. 33: 17-24). Since liposomes can be formed of almost any lipid a large variety of different liposomal formulations are known in the art. However, very little is known about the influence that individual lipids will have on the efficacy of a drug for a given disease let alone which molar ratios of two or more different lipids will lead to lipid compositions with an improved efficacy and/or an increased immune response.

It has been described in the prior art to administer antigens and adjuvants in a liposomal composition. Li et al. (2003) Vaccine, 21: 3319, describe the use of liposomes comprising phosphatidylcholine (PC) and cholesterol (CH) in equimolar amounts with and without 5 mol% phosphatidylethanolamine (PE), which further comprise CpG oligonucleotide and a HER-2/neu derived peptide. This formulation showed an increased immunization, if compared to the use of the free antigen.

Mui et al. (2001) J. Pharma. Exp. Therap., 298: 1185 disclose the increase of immune stimulation observed for free CpG ODN when encapsulated in liposomes comprising DSPC, CH, DODAP and myristoylsphingosine in a molar ratio of 20:45:25:10.

Lee et al. (1992) Biochimica Biophysica Acta, 1103: 185 studied the recognition of cells by liposomes, i.e. a cell type specificity of the liposomes, and the influence of specific lipid headgroups and surface charge densities on the recognition. They concluded that the presence of either up to 9 mol% phosphatidylserine (PS), phosphatidylglycerol (PG) or phosphatidic acid (PA) did increase the binding of liposomes, which otherwise comprised PC and cholesterol in a molar ratio of 2:1, to African monkey kidney cells. However, they also described that a further increase of either PS, PG or PA did not yield an improvement of specific binding to these cells.

Ludewig et al. (2000) Vaccine 19: 23 describe the *in vivo* antigen loading and activation of DC via a liposomal peptide vaccine and the resulting antiviral and anti-tumor immunity. The liposomes employed comprise a mixture of PC and cholesterol in a molar ratio of 5:1 and an immunostimulatory oligonucleotide.

Agrita et al. (2003) Infection and Immunity 71: 5210 describe targeting strategies for improving the interaction of liposomes with DC. The liposomes employed comprised PC, PS and cholesterol in a molar ratio of 8:2:2. or PC:PG:cholesterol in the same molar ratio. An enhanced immune response towards the vaccine was shown.

However, the factors influencing the suitability of a given liposome as a delivery vehicle, in particular to deliver antigens to cells of the hematopoietic cell lineage, which are involved in antigen presentation remain unclear. Thus there is still a need for delivery vehicles, which provide an improved efficacy, in particular an improved delivery to cells of the hematopoietic lineage. Such an improved delivery is particular for the administration of molecules, which stimulate and/or elicit an immune response like, for example, antigens and adjuvants.

### Detailed Description of the Invention

The present inventors have discovered that a liposome comprising cholesterol (CH) within a certain range and which additionally comprises two negatively charged lipids, i.e. PS and PG within certain ranges, or which comprises one negatively charged lipid, i.e. PS or PG, and PE within certain ranges, show an increased binding to cells of the hematopoietic lineage, in particular dendritic and Langerhans and/or elicit an enhanced immune response, if compared to prior art liposomes comprising no or only PS, PG or PE together with CH.

Therefore, one aspect of the present invention is the provision of a liposome, comprising in relation to the total molar lipid composition of the liposome:
a) CH between 20 mol% and 60 mol%; and
b) at least two components selected from the group of PS, between 20 mol% and 50 mol%, PG between 20 mol% and 50 mol%; and PE between 20 mol% and 50 mol%
c) at least one therapeutic agent, and/or at least one diagnostic agent.

It has been found that a CH concentration of above 60 mol% in the context of the other lipids is detrimental to the formation of regular lipid bilayer structures and, therefore, a content of 60% CH is the upper limit for the liposomes of the present invention. On the other hand lowering the cholesterol concentration below 20 mol% appears to increase the rate of elimination of the liposomes form the circulation and, thus, decreases the biological half life of a given therapeutic compound. In a preferred embodiment CH is present in relation to the total molar lipid composition of the liposome at a molar ratio of about 23 to about 42 mol%, more preferably about 26 to about 39 mol%, even more preferably about 30 to about 36 mol% and most preferably about 32 to about 34 mol%.

In a preferred embodiment of the liposome of the present invention PS is present in relation to the total molar lipid composition of the liposome at a molar ratio of about 23 to about 42 mol%, more preferably about 26 to about 39 mol%, even more preferably about 30 to about 36 mol% and most preferably about 32 to about 34 mol%.

In a preferred embodiment of the liposome of the present invention PG is present in relation to the total molar lipid composition of the liposome at a molar ratio of about 23 to about 42 mol%, more preferably about 26 to about 39 mol%, even more preferably about 30 to about 36 mol% and most preferably about 32 to about 34 mol%.

In a preferred embodiment of the liposome of the present invention PE is present in relation to the total molar lipid composition of the liposome at a molar ratio of about 23 to about 42 mol%, more preferably about 26 to about 39 mol%, even more preferably about 30 to about 36 mol% and most preferably about 32 to about 34 mol%.

In a particular preferred embodiment the liposome comprises in relation to the total molar lipid composition of each of CH and PS and PG about 23 to about 42 mol%, more preferably about 26 to about 39 mol%, even more preferably about 30 to about 36 mol% and most preferably about 32 to about 34 mol%.

In a particular preferred embodiment the liposome comprises in relation to the total molar lipid composition of each of CH and PS and PE about 23 to about 42 mol%, more preferably about 26 to about 39 mol%, even more preferably about 30 to about 36 mol% and most preferably about 32 to about 34 mol%.

In a particular preferred embodiment the liposome comprises in relation to the total molar lipid composition of each of CH and PG and PE about 23 to about 42 mol%, more preferably about 26 to about 39 mol%, even more preferably about 30 to about 36 mol% and most preferably about 32 to about 34 mol%.

The remainder of the lipid, i.e. the amount of lipid which is neither CH, PS and PG; CH, PS and PE; or CH, PG and PE, as the case may be, and which is needed to add up to 100 mol% can be made up of any lipid. The term "lipid" as used here and throughout the present invention refers to any substance having fatty or fat-like properties. In general a lipid comprises an extended apolar residue (X) and usually a water soluble, polar, hydrophilic residue (Y), which can be characterized by the basic formula

X-Yₙ

Wherein n equals or is greater than zero. Lipids with n = 0 are termed "apolar lipids", while lipids with n ≥ 1 a referred to as "polar lipids". Preferred lipids, which can make up the remainder of the lipids in the liposomes of the present invention are selected from the group consisting of glycerides, glycerophospholipides, glycerophosphinolipids, glycerophosphonolipids, sulfolipids, sphingolipids, phospholipids, isoprenolides, steroids, stearines, steroles and carbohydrate containing lipids.

Out of these lipids the remainder of the lipids preferably comprises one or more phospholipids. Preferably the phospholipid is selected from the group consisting of PC, and PE.

In a preferred embodiment of the liposome of the present invention comprise CH, PG and PS in above indicated ranges and preferred ranges further comprises PE in relation to the total molar lipid composition at a concentration of about 1 to about 40 mol%, preferably at about 5 to about 20 mol%, more preferably at about 8 to about 15 mol%.

In a further embodiment the liposome comprises PC in relation to the total molar lipid composition at a concentration of about 20 to about 40 mol%, preferably at about 5 to about 20 mol%, more preferably at about 8 to about 15 mol%.

In a particular preferred embodiment the lipids of the liposome of the present invention essentially consist of CH, PS and PG; CH, PS and PE or CH, PG and PE. In this case CH, PS, PG and/or PE can be present in their preferred and particularly preferred concentration ranges indicated above. Thus, in preferred embodiments the liposomes of the present invention essentially consists in relation to the total molar lipid composition out of CH and PS and PG, wherein each is present in a range of about 23 to about 42 mol%, more preferably about 26 to about 39 mol%, even more preferably about 30 to about 36 mol% and most preferably about 32 to about 34 mol%; out of CH and PS and PE, wherein each is present in a range of about 23 to about 42 mol%, more preferably about 26 to about 39 mol%, even more preferably about 30 to about 36 mol% and most preferably about 32 to about 34 mol%; out of CH and PG and PE, wherein each is present in a range of about 23 to about 42 mol%, more preferably about 26 to about 39 mol%, even more preferably about 30 to about 36 mol% and most preferably about 32 to about 34 mol%.

PS and PG are collective terms for lipids sharing a similar phosphatidylserine and phosphatidylglycerol, respectively, head group. However, many different apolar residues can be attached to these head groups. Thus, PSs and PGs isolated from different natural sources vary substantially in the length, composition and/or chemical structure of the attached apolar residues and naturally occurring PS and PG usually is a mixture of PSs and PGs with different apolar residues. While all PS and PG mixtures or pure isolated or chemically synthesized PS and PG compounds tested so far provide a good immune response when incorporated in the indicated ranges or preferred ranges into a liposome of the present invention it has been observed by the present inventors that certain PS and PG types stimulate a particular strong immune response and, therefore, the PS employed in the liposomes of the present invention is preferably selected from the group consisting of palmitoyloleoylphosphatidylserine, palmitoyllinoeoylphosphatidylserine, palmitoylarachidonoylphosphatidylserine, palmitoyldocosahexaenoylphosphatidylserine, stearoyloleoylphosphatidylserine, stearoyllinoleoylphosphatidylserine, stearoyl-arachidonoylphosphatidylserine, stearoyldocosahexaenoylphosphatidylserine, dicaprylphosphatidylserine, dilauroylphosphatidylserine, dimyristoylphosphatidylserine, diphytanoylphosphatidylserine, diheptadecanoylphosphatidylserine, dioleoylphosphatidylserine, dipalmitoylphosphatidylserine, distearoylphosphatidylserine, dilinoleoylphosphatidylserine dierucoylphosphatidylserine, didocosahexaenoyl-phospahtidylserine, PS from brain, and PS from soy bean; particular preferred is dioleoylphosphatidylserine. The PG employed in the liposome of the present invention is preferably selected from the group consisting of palmitoyloleoylphosphatidylglycerol, palmitoyllinoleoylphosphatidylglycerol, palmitoylarachidonoylphosphatidylglycerol, palmitoyldocosahexaenoylphosphatidylglycerol, stearoyloleoylphosphatidylglycerol, stearoyllinoleoylphosphatidylglycerol, stearoylarachidonoylphosphatidylglycerol, stearoyldocosahexaenoylphosphatidylglycerol, dicaprylphosphatidylglycerol dilauroylphosphatidylglycerol, diheptadecanoylphosphatidylglycerol, diphytanoyl-phosphatidylglycerol, dimyristoylphosphatidylglycerol, dipalmitoylphosphatidylglycerol, dielaidoylphosphatidylglycerol, distearoylphosphatidylglycerol, dioleoylphosphatidylglycerol, dilinoeoylphosphatidylglycerol, diarachidonoylphosphatidylglycerol, docosahexaenoylphosphatidylglycerol, and PG from egg; in particular dioleoylphosphatidylglycerol.

Similar to PS and PG PE is also a generic term for lipids sharing the phosphatidylethanolamine head group. It has also been observed by the present inventors that certain PEs stimulate a particular strong immune response, if incorporated into the liposomes of the present invention, therefore, in a preferred embodiment the PE is selected from the group consisting of the PE is selected from the group consisting of palmitoyloleoylphosphatidylethanolamine, palmitoyllinoleoylphosphatidylethanolamine, palmitoylarachidonoylphosphatidylethanolamine, palmitoyldocosahexaenoylphosphatidylethanolamine, stearoyloleoylphosphatidylethanolamine, stearoyllinoleoylphosphatidylethanolamine, stearoylarachidonoylphosphatidylethanolamine, stearoyldocosahexaenoylphosphatidylethanolamine, dilauroylphosphatidylethanolamine, dimyristoylphosphatidylethanolamine, diphytanoylphosphatidylethanolamine, dipalmitoylphosphatidylethanolamine, diheptadecanoylphosphatidylethanolamine, distearoylphosphatidylethanolamine, dielaidoylphosphatidylethanolamine, diarachidonoylphosphatidylethanolamine, docosahexaenoylphosphatidylethanolamine, PE from bacteria, PE from heart, PE from brain, PE from liver, PE from egg, and PE from soybean., in particular 1,2-diacyl-sn-glycero-3-PE, 1-acyl-2-acyl-sn-glycero-3-PE, 1,2-dipalmitoyl-PE and/or 1,2-dilauroyl-sn-glycero-3-PE (DLPE).

In a preferred embodiment the therapeutic agent is selected from the group consisting of a drug, an adjuvant or an antigen. The coadministration of an antigen together with an adjuvant is particularly preferred, since the immune response elicited by the antigen can be enhanced by the presence of an adjuvant. The term "adjuvant" as used herein refers to substances, which when administered prior, together or after administration of an antigen increases the immune response to the antigen in comparison to the administration of the antigen alone. Thus, in a preferred embodiment the liposome of the present invention comprises at least one adjuvant and at least one antigen.

The term "antigen" as used throughout the specification refers to all substances that elicit an immune response against the antigen in an animal, including a human, upon administration. Such an immune response can be characterized by, for example, a humoral and/or a cell-mediated immune response, which is accompanied by B cell proliferation and antibody secretion, activation of monocytes and/or macrophages as estimated by cytokine secretion (e.g. IL-1, IL-6, TNFα), activation and differentiation of dendritic cells (DC) as estimated by specific expression and/or up-or downregulation of specific surface antigens (e.g. MHC-class II, CD80, CD86, CD83, CD40, DC-LAMP which are upregulated and antigens, e.g. mannose-receptor, DEC-205, DC-SIGN which are downregulated) and by antigen-specific T cells, characterized by their expression of CD4 or CD8 and release of cytokines (e.g. IFNγ) upon activation (restimulation) with the appropriate antigen, in particular the same peptide antigen, used for immune response induction. Drugs in some cases can also elicit an immune response, however, such substances are considered antigens and not drugs, if the detected immune response satisfies the below defined criteria for a tumor antigen. It is preferred that the antigens or fragments thereof are capable of MHC presentation and, therefore, capable to elicit a cell-mediated immune response. In preferred embodiments of the invention the antigen is a tumor antigen, a viral antigen, a fungal antigen, a bacterial antigen, an autoantigen or an allergen.

The term "tumor antigen" comprises all substances, which elicit an immune response against a tumor. Particular suitable substances are those which are enriched in a tumor cell in comparison to a healthy cell. These substances are preferably present within and/or are accessible on the outside of the tumor cell. If the tumor antigen is only present within a tumor cell, it will still be accessible for the immune system, since the antigen or fragments thereof will be presented by the MHC system at the surface of the cell. In a preferred aspect tumor antigen is almost exclusively present on and/or in the tumor cell and not in a healthy cell of the same cell type.

Suitable tumor antigens can be identified, for example, by analyzing the differential expression of proteins between tumor and healthy cells of the same cell type using a microarray-based approach (Russo et al., Oncogene. 2003, 22:6497-507), by PCR- or microarray-based screening for tumor specific mutated cellular genes (Heller, Annu. Rev. Biomed. Eng. 2002, 4:129-53) or by serological identification of antigens by recombinant expression cloning (SEREX; Tureci et al., Mol Med Today. 1997, 3:342-349 ). The skilled artisan is aware of a large number of substances which are preferentially or exclusively present on and/or in tumor cell, which include for example, oncogenes like, for example truncated epidermal growth factor, folate binding protein, melanoferrin, carcinoembryonic antigen, prostate-specific membrane antigen, HER2-neu and certain sugar chains like, for example, epithelial mucins.

Not all of the substances that are preferentially or exclusively present in and/or on a tumor cell will elicit a strong immune response, therefore, it is preferred that tumor antigens are selected to be included in the liposomes of the present invention, which elicit a strong immune response. Antigens eliciting a strong immune response will induce at least 1%, preferably at least 5%, more preferably at least 10% and most preferably at least 15% IFNγ-producing CD8+ T or CD4+ T cells isolated from mice previously immunized with the antigen, upon challenge with the antigen and/or will induce preferably at least 5%, and most preferably at least 15% of B-cells cells isolated from mice previously immunized with the antigen, upon challenge with the antigen to proliferate. Antigens fulfilling these criterions are candidates for use in therapeutic and/or prophylactic cancer vaccines.

In a particular preferred embodiment the tumor antigen is selected from the group consisting of T-cell-defined cancer-associated antigens belonging to unique gene products of mutated or recombined cellular genes, in particular cyclin-dependent kinases (e.g.

CDC2, CDK2, CDK4), p15^{Ink4b}, p53, AFP, β-catenin, caspase 8, p53, p21^{Ras} mutations, Bcr-abl fusion product, MUM-1 MUM-2, MUM-3, ELF2M, HSP70-2M, HST-2, KIAA0205, RAGE, myosin/m, 707-AP, CDC27/m, ETV6/AML, TEL/Aml1, Dekcain, LDLR/FUT, Pml-RARα, TEL/AMLI; Cancer-testis (CT) antigens, in particular NY-ESO-1, members of the MAGE-family (MAGE-A1, MAGE-A2, MAGE-A3, MAGE-A4, MAGE-A6, MAGE-10, MAGE-12), BAGE, DAM-6, DAM-10, members of the GAGE-family (GAGE-1, GAGE-2, GAGE-3, GAGE-4, GAGE-5, GAGE-6, GAGE-7B, GAGE-8), NY-ESO-1, NA-88A, CAG-3, RCC-associated antigen G250; Tumor virus antigens, in particular human papilloma virus (HPV)-derived E6 E7 oncoproteins, Epstein Barr virus EBNA2-6, LMP-1, LMP-2; overexpressed or tissue-specific differentiation antigens, in particular gp77, gp100, MART-1/Melan-A, p53, tyrosinase, tyrosinase-related protein (TRP-1 and TPR-2), PSA, PSM, MC1R; widely expressed antigens, in particular ART4, CAMEL, CEA, CypB, HER2/neu, hTERT, hTRT, iCE, Muc1, Muc2, PRAME RU1, RU2, SART-1, SART-2, SART-3, and WT1; and fragments and derivatives thereof. Particular preferred tumor antigens are antigens derived from the tyrosinase-related protein. The tumor antigen or fragments thereof, selected to be included in a liposome of the present invention preferably stimulates a cell-mediated immune response.

The term "viral antigen" comprises all substances, which elicit an immune response against a virus, in particular against a virally infected cell. It is preferred that the viral antigen elicits a strong immune response as defined above. In preferred embodiments of the present invention the viral antigen is derived from a virus selected from the group consisting of Retroviridae, in particular HIV-1 and HIV-LP; Picomaviridae, in particular polio virus and hepatitis A virus; enterovirus, in particular human coxsackie virus, rhinovirus, echovirus; Calciviridae, in particular strains that cause gastroenteritis; Togaviridae, in particular equine encephalitis virus and rubella virus; Flaviridae, in particular dengue virus, encephalitis virus and yellow fever virus; Coronaviridae, in particular coronavirus; Rhabdoviridae, in particular vesicular stomatitis virus and rabies virus; Filoviridae, in particular Ebola virus or and Marburg virus; Paramyxoviridae, in particular parainfluenza virus, mumps virus, measles virus and respiratory syncytical virus; Orthomyxoviridae, in particular influenza virus; Bungaviridae, in particular Hantaan virus, bunga virus, phlebovirus and Nairo virus; Arena viridae, in particular hemorrhagic fever virus; Reoviridae, in particular reovirus, orbivirus and rotavirus; Bimaviridae; Hepadnaviridae, in particular Hepatitis B virus; Parvovirida, in particular parvovirus; Papovaviridae, in particular papilloma virus, simian virus-40 (SV40) and polyoma virus; Adenoviridae; Herpesviridae, in particular herpes simplex virus (HSV) 1 and 2, varicella zoster virus, cytomegalovirus (CMV), herpes virus; Poxviridae, in particular variola virus, vaccinia virus and pox virus; and Iridoviridae, in particular African swine fever virus; and Hepatitis C. Particularly preferred viral antigens are selected from the group consisting of HPV L6, HPV L7, fragments and derivatives thereof. The viral antigens or fragments thereof, which can be comprised in the liposomes of the present invention, preferably stimulate a cell-mediated immune response.

The term "fungal antigen" comprises all substances, which elicit an immune response against a fungus. It is preferred that the fungal antigen elicits a strong immune response as defined above. In preferred embodiments of the present invention the fungal antigen is derived from a fungus selected from the group consisting of *Cryptococcus* species, in particular *Cryptococcus neoformans, Histoplasma* species, in particular *Histoplasma capsulatum, Coccidioides* species, in particular *Coccidioides immitis, Blastomyces* species, in particular Blastomyces *dermatitidis, Chlamydia* species, in particular *Chlamydia trachomatis,* and *Candida* species, in particular *Candida albicans*. The fungal antigens or fragments thereof, which are preferably comprised in the liposomes of the present invention stimulate a humoral immune response.

The term "bacterial antigen" comprises all substances, which elicit an immune response against a bacterium. It is preferred that the bacterial antigen elicits a strong immune response as defined above. In preferred embodiments of the present invention the bacterial antigen is derived from a bacterium selected from the group consisting of Helicobacter species, in particular *Helicobacter pyloris*; Borelia species, in particular *Borelia burgdorferi;* Legionella species, in particular *Legionella pneumophilia*; Mycobacteria species, in particular *M. tuberculosis, M. avium, M. intracellulare, M. kansasii, M. gordonae;* Staphylococcus species, in particular *Staphylococcus aureus*; Neisseria species, in particular *N. gonorrhoeae, N. meningitidis;* Listeria species, in particular *Listeria monocytogenes;* Streptococcus species, in particular *S. pyogenes, S. agalactiae*; *S. faecalis; S. bovis, S. pneumoniae*; anaerobic Streptococcus species; pathogenic Campylobacter species; Enterococcus species; Haemophilus species, in particular *Haemophilus influenzae*; Bacillus species, in particular *Bacillus anthracis*; Corynebacterium species, in particular *Corynebacterium diphtheriae*; Erysipelothrix species, in particular *Erysipelothrix* *rhusiopathiae*; Clostridium species, in particular *C. perfringens, C. tetani;* Enterobacter species, in particular *Enterobacter aerogenes*, Klebsiella species, in particular *Klebsiella pneumoniae*, Pasturella species, in particular *Pasturella multocida,* Bacteroides species; Fusobacterium species, in particular *Fusobacterium nucleatum;* Streptobacillus species, in particular *Streptobacillus moniliformis;* Treponema species, in particular *Treponema pertenue;* Leptospira; pathogenic Escherichia species; and Actinomyces species, in particular *Actinomyces israelli.* The bacterial antigens or fragments thereof, preferably comprised in the liposomes of the present invention stimulate a humoral immune response.

The term "autoimmune antigen" comprises all substances, which elicit an immune response against a substance, e.g. a protein, which is normally present in the body, in particular in a healthy cell, tissue, or organ. Autoimmune antigens can be used for desensitization strategies for the treatment and/or prevention of autoimmune diseases like, for example, type 1 diabetes, conventional organ-specific autoimmune diseases, neurological diseases, rheumatic diseases, psoriasis, connective tissue diseases, autoimmune cytopenias, and other autoimmune diseases. Such conventional organ specific autoimmunity may include thyroiditis (Graves+Hashimoto's), gastritis, adrenalitis (Addison's), ovaritis, primary biliary cirrhosis, myasthenia gravis, gonadal failure, hypoparathyroidism, alopecia, malabsorption syndrome, pernicious anemia, hepatitis, anti-receptor antibody diseases and vitiligo. Such neurological diseases may include schizophrenia, Alzheimer's disease, depression, hypopituitarism, diabetes insipidus, sicca syndrome and multiple sclerosis. Such rheumatic diseases/connective tissue diseases may include rheumatoid arthritis, systemic lupus erythematous (SLE) or Lupus, scleroderma, polymyositis, inflammatory bowel disease, dermatomyositis, ulcerative colitis, Crohn's disease, vasculitis, psoriatic arthritis, exfoliative psoriatic dermatitis, pemphigus vulgaris, Sjogren's syndrome. Other autoimmune related diseases may include autoimmune uvoretinitis, glomerulonephritis, post myocardial infarction cardiotomy syndrome, pulmonary hemosiderosis, amyloidosis, sarcoidosis, aphthous stomatitis, and other immune related diseases, as presented herein and known in the related arts. Autoimmune antigens responsible for the respectively indicated diseases are known in the art and can all without limitation be comprised in a liposome of the present invention.

The term "allergen" refers to a substance, which elicits an immune response against an extraneous substance, which is not a viral, bacterial or fungal antigen. Allergens can be comprised in the liposomes of the present invention for treatment or prevention of allergies by a desensitization strategy. Preferred allergens are selected or derived from the group consisting of pollen, in particular from maple, birch, alder, hazelnut, mugwort, beach mountain cedar, oak, walnut, elm, olive, sycamore, cottonwood, white ash, and white pine; grass, in particular from sweet vernal grass, orchard grass, Bermuda grass, oat grass, rye grass; insects, in particular mites; food stuff, in particular milk and milk products, nuts, in particular peanuts, hazelnut and almonds; animal hair, in particular hair derived from cat, horse, donkey, sheep, goat, dog, mice, rat, guinea pig, and rabbit. Further allergens, which can be included in the liposomes of the present invention are allergens, which elicit contact hypersensitivity like, for example, nickel and copper.

The liposomes of the present invention show an increased affinity towards certain cell types of the body, i.e. cells of the hematopoietic lineage, and can thus be used for the delivery of drugs preferentially to these cells types. In a preferred embodiment the drug is selected from the group consisting of analgesics; antirheumatics; anthelminthics; antiallergics; antianemics; antiarrhythmics; antibiotics; angiogenesis inhibitors; antiinfectives; antidemenics (nootropics); antidiabetics; antidotes; antiemetics; antivertiginosics; antiepileptics; antihemorrhagics; antihypertonics; antihypotonics; anticoagulants; antimycotics; antitussive agents; antiviral agents; beta-receptor and calcium channel antagonists; broncholytic and antiasthmatic agent; chemokines; cytokines, in particular immune modulatory cytokines; mitogens; cytostatics; cytotoxic agents and prodrugs thereof; dermatics; hypnotics and sedatives; immunosuppressants; immunostimulants in particular activators of NF-κB, MAP kinases, STAT proteins and/or protein kinase B/Akt; peptide or protein drugs; in particular hormones and physiological or pharmacological inhibitors of mitogens, chemokines, or cytokines or their respective prodrugs. Of course it is also envisioned that a liposome of the invention comprises more than one drug at once or comprises one or more drugs together with one or more antigens and/or one or more adjuvants. In a preferred embodiment the drug is selected from the group consisting of chemokines, cytokines, mitogens, cytostatics, cytotoxic agents and prodrugs thereof, immunostimulants, peptide or protein drugs, in particular hormones and physiological or pharmacological inhibitors of mitogens, chemokines, or cytokines or their respective prodrugs.

As the liposomes of the present invention show a marked cell type specificity they can also be used to deliver a diagnostic agent preferentially to that particular tissue. It is also envisioned that the diagnostic agent is comprised together with therapeutic agent, which would allow monitoring of the delivery and distribution of the therapeutic agent with a patient. It is even more preferred that the liposome comprises a diagnostic agent, if the liposome comprises a targeting moiety as defined below, which further aides the preferential localization of the liposomes of the present invention in the targeted tissues or disease sites.

The term "diagnostic agent" refers to a chemical moiety which is directly or indirectly detectable by analytical methods including measurement of fluorescence, nuclear magnetic resonance, computer tomography or scintigrams. In preferred embodiments the diagnostic agent is selected from the group consisting of an electron dense molecule, a paramagnetic molecule, a superparamagnetic molecule, a radioactive molecule, a non-radioactive isotope, and a fluorescent molecule like, for example, ¹³N, ¹⁵O, ¹⁸F, ⁵¹Gr, ⁵⁴Fe, ⁶⁰Co, ⁶⁷Ga, ⁷⁵Se, ^{99m}Tc, ¹¹¹In, ^{112m}Ag, ^{113m}In, ¹²³I, ¹³³Xe, ¹⁴⁸Au, ³⁵S, ³³P, ³²P, or ¹¹C, non-radioactive isotopes, which include, for example, ²H and ¹³C, and fluorescent molecules or molecules generating fluorescence or light emission like, for example, green fluorescent protein, luciferase, and a variety of fluorescent dies all of which are well known to someone of skill in the art.

As previously outlined the liposomes of the present invention have an increased affinity for certain hematopoietic cell lineages, more specifically for dendritic cells and Langerhans cells. While this specificity makes the liposomes of the present invention particular suitable to deliver antigens and/or adjuvants in immunization/vaccination strategies, which aim at the prevention or treatment of a variety of diseases, this property renders the liposomes particular suitable as delivery vehicles to deliver drugs to cells of the hemopoietic cell lineage. The liposome can comprise any cytostatic or cytotoxic drug, however, from the known cytostatic and cytotoxic drugs the following are particularly preferred: alkylating substances, anti-metabolites, antibiotics, epothilones, nuclear receptor agonists and antagonists, anti-androgens, anti-estrogens, platinum compounds, hormones and antihormones, interferons and inhibitors of cell cycle-dependent protein kinases (CDKs), inhibitors of cyclooxygenases and/or lipoxygenases, biogenic fatty acids and fatty acid derivatives, including prostanoids and leukotrienes, inhibitors of protein kinases, inhibitors of protein phosphatases, inhibitors of lipid kinases, platinum coordination complexes, ethyleneimenes, methylmelamines, trazines, vinca alkaloids, pyrimidine analogs, purine analogs, alkylsulfonates, folic acid analogs, anthracendiones, substituted urea, methylhydrazin derivatives, in particular acediasulfone, aclarubicine, ambazone, aminoglutethimide, L-asparaginase, azathioprine, bleomycin, busulfan, calcium folinate, carboplatin, carpecitabine, carmustine, celecoxib, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, enediynes, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon α, irinotecan, leuprolide, lomustine, lurtotecan, mafenide sulfate olamide, mechlorethamine, medroxyprogesterone acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, preussin, procarbazine, pyrimethamine, raltitrexed, rapamycin, rofecoxib, rosiglitazone, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, staurosporin, tamoxifen, taxol, teniposide, tertiposide, testolactone, testosteronpropionate, thioguanine, thiotepa, tinidazole, topotecan, triaziquone, treosulfan, trimethoprim, trofosfamide, UCN-01, vinblastine, vincristine, vindesine, vinblastine, vinorelbine, and zorubicin, or their respective derivatives or analogs thereof. Several of the above indicated drugs are now administered simultaneously for cancer therapy and, consequently, it is also envisioned that more than one cytostatic and/or cytotoxic drug is comprised in a liposome of the present invention.

The present inventors have discovered that the liposome of the present invention can elicit substantial immune responses against antigens comprised in or attached to the liposome.

Albeit it is known in the art that an immune response can be stimulated even further when an adjuvant is co-administered with an antigen it was surprisingly found that liposomes comprising an adjuvant led to a much stronger immune stimulation, if compared to the coadministration of "free" adjuvant. Thus, in a preferred embodiment the liposome of the present invention comprises at least one adjuvant. The adjuvant can be comprised "freely" within the interior of the liposome or it can be attached to any component making up the liposome like, for example, a lipid, preferably, PE, PS and/or PG, a protein comprised within the liposome or integrated into the membrane of the liposome. In some embodiments, in which the liposome of the present invention comprises both an antigen and an adjuvant the adjuvant can also be attached to the antigen.

Preferably the adjuvants, which can be comprised in the liposomes of the present invention increase the response to an antigen at least by 20%, preferably at least by 50%, more preferably at least by 100%, more preferably at least by 200% and most preferably by at least a 1000%. Preferred adjuvants are selected from the group consisting of unmethylated DNA, in particular unmethylated DNA comprising CpG dinucleotides (CpG motif), in particular CpG ODN with phosphorothioate (PTO) backbone (CpG PTO ODN) or phosphodiester (PO) backbone (CpG PO ODN); gel-like precipitates of aluminum hydroxide (alum); bacterial products from the outer membrane of Gram-negative bacteria, in particular monophosphoryl lipid A (MPLA), lipopolysaccharides (LPS), muramyl dipeptides and derivatives thereof; synthetic lipopeptide derivatives, in particular Pam₃Cys; lipoarabinomannan; peptidoglycan; zymosan; heat shock proteins (HSP), in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, interleukin- (IL-)2, IL-6, IL-7, IL-18, type I and II, interferons, in particular interferon-gamma, TNF-alpha; oil in water emulsions, in particular MF59 consisting of squalene; Tween 80 and Span 85 (sorbitan-trioleate) and QS-21, a more highly purified derivative of Quil A, non-ionic block polymers, in particular Poloxamer 401, saponins and derivatives thereof, in particular the immunostimulatory fragments from saponins; polyphosphazene; N-(2-Deoxy-2-L-leucylamino-β-D-glucopyranosyl)-N-octadecyldodecanoylamide hydroacetate (BAY R1005), 25-dihydroxyvitamin D3 (calcitriol); DHEA; murametide [MDP(Gln)-OMe]; murapalmitine; polymers of lactic and/or glycolic acid; polymethyl methacrylate; sorbitan trioleate; squalane; stearyl tyrosine; squalene; theramide, synthetic oligopeptides, in particular MHCII-presented peptides. Particular preferred adjuvants, which can be comprised in the liposome of the present invention are selected from the group unmethylated DNA, in particular unmethylated DNA comprising CpG dinucleotides (CpG motif), in particular CpG ODN with phosphorothioate (PTO) backbone (CpG PTO ODN) or phosphodiester (PO) backbone (CpG PO ODN) and synthetic lipopeptide derivatives, in particular Pam₃Cys.

The liposome of the present invention can comprise two or more adjuvants. Preferably the two or more adjuvants will act synergistically in the stimulation of the immune system. A synergistic effect of two adjuvants can in particular be observed, if the adjuvants stimulate different molecular pathways involved in the mediation of the adjuvant effect. Thus, if two or more adjuvants are used it is preferred that each stimulates the immune response through a different pathway. Pathways, which stimulate an immune response are known to the skilled artisan and comprise, for example, the Toll-like receptor 2 (TLR2), TLR 3, TLR4, TLR5, TLR7 and TLR9 pathway. It is therefore preferred, that two adjuvants are comprised in the liposome that stimulate different TLR pathways. The skilled artisan knows adjuvants, which stimulate the respective TLR pathways. For TLR2 such adjuvants include, for example, lipopeptides, lipoarabinomannan, peptidoglykan, zymosan and HSPs; for TLR3, for example, DS-RNA; for TLR4, for example, lipopolysaccharid, HSPs, Taxol, RSV, fibronectin; for TLR5, for example, flagellin; for TLR7, for example, imidazoquinoline; and for TLR9, for example, unmethylated DNA, in particular CpG-DNA; unmethylated phosphorothioate (PTO) oligonucleotides, in particular CpG-PTO oligonucleotides.

Preferably the liposome of the present invention comprise at least one antigen, preferably at least one tumor antigen, and at least one and more preferably at least two adjuvants. In this context the adjuvants are preferably selected to act synergistically as outlined above.

In preferred embodiments the liposome of the present invention comprises the therapeutic agent and/or the diagnostic agent in an amount such that the ratio of the molar amount of therapeutic and/or diagnostic agent to the molar amount of total lipids is between 1:100 and 1:10, preferably between 1:80 and 1:15 and more preferably between 1:50 and 1:20.

Liposomes of the present invention can have a diameter between 10 and 1000 nm. They, however, have in a preferred embodiment a diameter of between 50 and 200 nm and more preferably between 80 and 150 nm. The diameter of the liposomes can be affected, for example, by extrusion of the liposomal composition through sieves or meshes with a known pore size. This and further methods of controlling the size are well known in the art and are described, for example, in Mayhew et al. (1984) Biochim. Biophys. Acta 775:169-174 or Olson et al. (1979) Biochim. Biophys. Acta 557:9-23.

In a further embodiment any of the components making up the membrane of the liposomes of the present invention can be attached to a further chemical moiety. The term chemical moiety is not particular limited. However, in preferred embodiments the chemical moiety is a targeting moiety as discussed in more detail below or a stabilizing moiety.

The term "attached" as used throughout this description refers to a direct or indirect, covalent or non-covalent bond and connection, respectively, between a chemical moiety in particular a targeting moiety or stabilizing moiety and another component of the liposome. A wide variety of chemical groups which allow attachment as defined above are known in the art including, for example, biotin-streptavidin, amino-reactive groups (e.g. carbodiimides, hydroxylmethylphosphine, imidoester, N-hydroxysuccinimide esters, isothiocyanates, isocyanates), sulfhydryl-reactive groups (e.g. maleimides, haloacetyls, pyridyl disulfides, aziridines) carboxyl-reactive molecules (e.g. carbodiimides, carbodiimidazole, diaoalkanes), hydroxyl-reactive groups (e.g. carbonyldiimidazole, alkyl halogens, isocyanates), and can readily be selected by someone of skill in the art as appropriate.

Stabilizing moieties within the meaning of this invention increase the circulation time of the liposome once it is administered. Particular preferred stabilizing moieties are ganglioside GM1, phosphatidylinositol or PEG, particular preferred PEGs have a molecular mass between about 1,000 and about 10,000 g/mol, more preferably about 5,000 g/mol.

In a preferred embodiment the chemical moieties in particular the stabilizing moieties are attached to only a fraction of the molecules making up the membrane of the liposomes. It is preferred that between about 1 to about 20 mol% of the components of the liposomal membrane carry an attached chemical moiety, more preferably between about 3 and about 10 mol% and even more preferably about 5 mol%.

A preferred liposomal component for attachment of the chemical moiety, in particular for the stabilizing moiety is a lipid component. While different chemical moieties can be attached to different lipid components it is preferred that the chemical moiety(ies) is(are) attached to one or more of the phospholipids comprised within the liposome of the present invention. In a further preferred embodiment the one or more chemical moiety is attached to PE. In particular, if a stabilizing agent like, for example, PEG is used PE is used for attachment.

In addition to the attachment of stabilizing moieties detergents, proteins and peptides can be incorporated into the liposome for stabilizing the lipid bilayers of the liposomes of the present invention. Detergents which can be used as bilayer stabilizing components include, but are not limited to, Triton X-100, deoxycholate, octylglucoside and lysophosphatidylcholine. Proteins which can be used as bilayer stabilizing components include, but are not limited to, glycophorin and cytochrome oxidase. In preferred embodiments a liposome can comprise between 0.05 and 15 mol% of a stabilizing agent.

As pointed out above the liposomes of the present invention exhibit a preference for binding to certain cells, in particular to cells of the hematopoietic lineage. In some applications like, for example, in vaccination strategies it can be desirable that an antigen is even more specifically delivered to cells of the hematopoietic lineage. This can be achieved by providing the liposomes with a means of targeting, which allows targeting of the liposomes primarily to a specific site with in the body, which can aid in decreasing unwanted systemic effects and/or toxicity. Therefore, in a further embodiment of the liposomes of the present invention a targeting moiety is attached to the liposome. As outlined above with respect to the chemical moiety the targeting moiety can be attached to any component of the liposome. Preferably, the targeting moiety is: a) attached to one of the lipid components of the liposome, b) attached to a membrane protein which can be incorporated into the membrane of the liposomes of the present invention or c) is itself capable of insertion or integration in the lipid layer.

In a preferred embodiment the targeting moiety is selected from the group consisting of a peptide or protein, in particular an antibody or fragment thereof, a single-chain antibody or fragment thereof, a receptor ligand or fragment thereof; a carbohydrate; and a ligand.

More specifically the targeting moiety can be selected from the group consisting of natural or synthetic receptor-binding peptides and mimetics thereof, mono- or oligosaccharides, receptor ligands or fragments thereof, antibodies or fragments thereof, all of which are directed against DC-specific surface molecules or receptors, in particular CD54 (ICAM-1) and ICAM-2, mannose receptor, CD207 (langerin), ASGPR, CLEC-1, CLEC-2, DCIR, dectin-1, DC-SIGN, DEC-205, BDCA-2, TLR-1, TLR-2, TLR-3, TLR-4, TLR-5, TLR-7, TLR-9, CD40, CD16/32 (FcγR-III and -II), CD11, CD1a, CDId, and MHC class II.

In a preferred embodiment the targeting moiety is attached to a spacer. The term "spacer" as used throughout the description refers to a chemical moiety, which serves the purpose of providing better accessibility of the targeting moiety even when it is attached to a component of the liposome, e.g. a lipid, which might otherwise sterically hinder the binding of the targeting moiety to its respective target structure. Spacers within this meaning have a linear extension of at least 0.5 nm preferably the spacer has a linear extension of between 1 and 10 nm and even more preferably between 2 and 5 nm. The spacer is preferably a linear or branched saturated or unsaturated carbohydrate chain. The carbohydrate chain preferably comprises multimeric repeats of a monomeric building block. Depending on the length of the respective monomeric building block between 2 and 10 multimeric repeats of the monomeric building blocks are preferred. In preferred embodiments the spacer is hydrophilic. The spacer can comprise a functional group which allows attachment to the targeting moiety on one terminus and another functional group on the other terminus, which allows attachment of the spacer to a component of the liposome, e.g. a lipid of the present invention.

Preferred spacers are bifunctional molecules, in particular, bifunctional polyethylene or polypropylene glycol derivatives comprising preferably between about 1 and 40 repeat units, oligopeptides comprising natural and/or synthetic amino acids. The oligopeptides preferably comprise between 1 and 40, preferably between 2 and 20 and more preferably between 2 and 10 amino acids. A particular preferred building block of a spacer is 8-amino-3, 6-dioxatanoic acid (doo) and spacers comprising between 1 to 10 repeat units of doo are preferred. Spacers comprising between 2 and 5 doo units are even more preferred and spacers comprising 3 doo units are most preferred. In the context of liposomes it has been discovered by the present inventors that there is an optimal length of the spacer, which is between 2 and 5 nm. On one hand spacers with a length of less than about 0.5 nm will in most cases not provide enough distance from the liposomal surface to which the targeting moiety has been attached to allow efficient interaction, i. e. binding, between the targeting moiety and its respective target like, for example, a tumor cell. On the other hand spacers, which are longer than about 10 nm show an increasing "floppiness", which is also detrimental to the interaction between the targeting moiety and its target. Thus, in a preferred embodiment the spacer has a length of between about 1 and about 10 nm, preferably between about 2.5 and about 5 nm.

In preferred embodiments of liposomes of the present invention the targeting moiety is attached to a lipid, preferably a phospholipid like, for example PE, PG, PC or PS and preferably the lipid, which is used for attachment of a targeting moiety is selected from the group consisting of N-caproylamine-PE, N-dodecanylamine-PE, phophatidylthioethanol, N-[4-(p-maleimidomethyl)cyclohexane-carboxamide-PE (N-MCC-PE), N-[4-(p-maleimidophenyl)butyramide]-PE (N-MPB), N-[3-(2-pyridyldithio)propionate]-PE (N-PDP), N-succinyl-PE, N-glutaryl-PE, N-dodecanyl-PE, N-biotinyl-PE, N-biotinyl-cap-PE, phosphatidyl-(ehtylene glycol), PE-polyethylene glycol (PEG)-carboxylic acid, PE-PEG-maleimide, PE-PEG-PDP, PE-PEG-amine, PE-PEG-biotin, PE-PEG-HNS, dipalmitoylglycerosuccinyl-lysine, alpha-methoxy-omega-(1,2-dioctadecenoyloxy glyceryl) (DO), alpa-methoxy-omega-(1,2-ditetradecenoyloxy glyceryl) (DT).

As outlined above the main components and in many embodiments the only components making up the membrane of the liposome of the present invention are lipids. However, in some aspects of the present invention the membrane of the liposomes can further comprise components, which are capable of insertion/integration into the lipid layer. Examples of such components are proteins with a hydrophilic portion, including one or more membrane spanning domains or GPI-anchors, or other amphipathic molecules such as lipopeptides and glycolipids or molecules conjugated or fused to one or more fatty acid, lipid or other hydrophobic moieties. Such molecules can, for example, provide the liposome with a targeting capacity, i.e. can be a targeting moiety as defined above, or can have an enzymatic function.

Since it has been discovered by the present inventors, that the presence of the negatively charged components PS or PG in the context of PE and in preferred embodiments the presence of both PS and PG leads to liposomes, which elicit a stronger immune response than prior art liposomes it is preferred that the net surface charge of the liposome is negative, i.e. that the liposome comprises an amount of negatively charged lipids, which exceeds the amount of positively charged lipids in the liposome.

The liposomes of the present invention are stable structures, which can, for example, be filtered after production to remove surrounding drug solutions or buffers. The "pure" liposomes with or without therapeutic agent and/or diagnostic agent can be used, however, due to its stability it is also possible to remove essentially all liquid from the liposome to facilitate easy storage in a dried state. Therefore, the liposomes of the present invention can be supplied in dried form, preferably in a freeze dried form. These liposomes can be readily rehydrated upon addition of water alt solution and/or buffer at the time and point of use.

It is particularly preferred that the therapeutic or diagnostic compound is comprised in the interior of the liposome or in cases of lipophilic drugs also within or between the lipid bilayers. A variety of methods are available in the prior art to "load" a liposome with a given therapeutic and/or diagnostic agent. In its simplest form the therapeutic or diagnostic agent(s) is(are) admixed with the lipid components during formation of the liposomes. Other passive loading methods include dehydration-rehydration (Kirby & Gregoriadis (1984) Biotechnology 2:979), reverse-phase evaporation (Szoka & Papahadjopoulos (1978) Proc. Natl.Acad. Sci. USA 75:4194-), or detergent-depletion (Milsmann et al. (1978) Biochim. Biophys. Acta 512:147-155). However, these techniques often lead to a substantial loss of therapeutic and/or diagnostic agent during loading, which is a particular disadvantage in cases where the therapeutic or diagnostic agent is expensive.

Other methodologies for encapsulating therapeutic and/or diagnostic agents include so called "remote loading" or "active loading" in which due to a gradient, for example, a pH or salt gradient between the exterior and the interior of a preformed liposome the therapeutic or diagnostic agent is transported into the liposome along the gradient (see, for example, Cheung et al. (1998) Biochim. Biophys. Acta 1414:205-216; Cullis et al. (1991) Trends Biotechnol. 9:268-272; Mayer et al. (1986) Chem. Phys. Lipids 40:333-345).

Most active and passive loading procedures require the solubilization of the therapeutic and/or diagnostic compound in a solvent. For some compounds in particular hydrophobic compounds or compounds of higher molecular weight, like, for example, peptides or proteins the solubilization in an aqueous solvent can proof difficult and this can make loading inefficient and, thus, in particular with expensive compounds uneconomical. Thus, rather than using an aqueous solvent in those cases organic solvents have been used in the prior art. However, the administration of liposomes or liposomal compositions comprising organic solvents is often not feasible since they pose biocompatibility problems and, therefore, the organic solvents have to be removed prior to administration. The present inventors, however, have now discovered that the liposomes of the present invention can efficiently be produced with a method comprising the steps of:
a) forming a suspension of lipids comprising CH in above indicated molar ranges and preferred ranges, and at least two components selected from the group consisting of PS, PG, and PE in above indicated molar ranges and preferred ranges and one or more therapeutic and/or diagnostic agent and a liquid medium and
b) homogenizing the suspension.

In a preferred embodiment the lipids and/or the therapeutic and/or the diagnostic agent are essentially not soluble in the liquid medium. Preferably the therapeutic and/or diagnostic agent is essentially not soluble. Preferred liquid mediums are H₂0, aqueous salt solutions and/or buffer solutions. Preferably the lipids and therapeutic and/or diagnostic agents are employed in above indicated ranges and preferred ranges.

Further passive and active loading techniques are well known in the art and can all without limitation be employed by the skilled artisan to produce the liposomes of the present invention. The most efficient method of loading for any given therapeutic and/or diagnostic compound can be determined by routine experimentations by well established procedures. Variables which are typically adjusted are pH, temperature, salt type and concentration, type of buffer etc.

In a preferred embodiment the therapeutic and/or diagnostic agent(s) is (are) loaded by remote loading into the liposomes, since this method offers a very low loss of the substance to be loaded. In a preferred embodiment a pH gradient is used for loading. Depending on the substance to be loaded the interior of the liposome will typically be acidified with respect to its exterior. Preferably the interior will have a pH between 1 and 6 prior to loading with the therapeutic and/or diagnostic agent.

Therefore, another aspect of the present invention is a liposome produced by one of the above methods, in particular the method of forming a suspension of lipids comprising CH, and at least one component selected from the group consisting of PS, PG, and PE one or more therapeutic and/or diagnostic agent and a liquid medium and homogenizing the suspension.

A further aspect of the present invention is a liposomal composition comprising the liposome of the present invention in a liquid medium, which comprises a substance selected from the group consisting of adjuvants, additives, buffers and auxiliary substances. The liquid medium is preferably a biocompatible aqueous medium like PBS, Ringer's solution or the like. Preferably the liquid medium comprises at least one adjuvant.

The term adjuvant in this context has the same meaning as outlined above. Preferred adjuvants, which can be comprised in a liposomal composition of the present invention are selected from the group consisting of unmethylated DNA comprising CpG dinucleotides (CpG motif), in particular CpG ODN with phosphorothioate (PTO) backbone (CpG PTO ODN) or phosphodiester (PO) backbone (CpG PO ODN); gel-like precipitates of aluminum hydroxide (alum); bacterial products from the outer membrane of Gram-negative bacteria, in particular monophosphoryl lipid A (MPLA), lipopolysaccharides (LPS), muramyl dipeptides and derivatives thereof; synthetic lipopeptide derivatives, in particular Pam₃Cys; lipoarabinomannan; peptidoglycan; zymosan; heat shock proteins (HSP), in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, interleukin-(IL-)2, IL-6, IL-7, IL-18, type I and II, interferons, in particular interferon-gamma, TNF-alpha; oil in water emulsions, in particular MF59 consisting of squalene; Tween 80 and Span 85 (sorbitan-trioleate) and QS-21, a more highly purified derivative of Quil A, non-ionic block polymers, in particular Poloxamer 401, saponins and derivatives thereof, in particular the immunostimulatory fragments from saponins; polyphosphazene; N-(2-Deoxy-2-L-leucylamino-β-D-glucopyranosyl)-N-octadecyldodecanoylamide hydroacetate (BAY R1005), 25-dihydroxyvitamin D3 (calcitriol); DHEA; murametide [MDP(Gln)-OMe]; murapalmitine; polymers of lactic and/or glycolic acid; polymethyl methacrylate; sorbitan trioleate; squalane; stearyl tyrosine; squalene; theramide, synthetic oligopeptides, in particular MHCII-presented peptides. Particular preferred adjuvants, which can be comprised in the liposome of the present invention are selected from the group unmethylated DNA, in particular unmethylated DNA comprising CpG dinucleotides (CpG motif), in particular CpG ODN with phosphorothioate (PTO) backbone (CpG PTO ODN) or phosphodiester (PO) backbone (CpG PO ODN) and synthetic lipopeptide derivatives, in particular Pam₃Cys.

The liposome comprised in the liposomal composition preferably comprises an antigen, in particular a tumor antigen, a viral antigen, a fungal antigen, a bacterial antigen an autoantigene or an allergen. In one embodiment of the inventive liposomal composition, wherein the liposome comprises at least one antigen, the liposomal composition comprises at least one adjuvant. This adjuvant can be comprised within the liposome and/or can be comprised within the liquid medium wherein the liposomes are comprised. Preferably at least one adjuvant is comprised both within the liposome and within the liquid medium, wherein the liposomes are comprised. It is further preferred that the adjuvants within and/or outside the liposome act synergistically to enhance immune stimulation. To achieve such a synergistic action it is preferred that the adjuvants act upon different molecular pathways, in particular upon different TLR pathways as outlined above. In addition it is preferred that the liposomal composition comprises outside of the liposome a cytokine with adjuvant activity like, for example, GM-CSF, IL-2, IL-6, IL-7, IL-18, type I and II, interferons, in particular interferon-gamma, or TNF-alpha. The use of GM-CSF is particularly preferred, i.e. a liposomal composition comprising GM-CSF outside the liposome and another adjuvant, preferably one of the above indicated preferred adjuvants inside the liposome.

The liposome of the present invention and/or the liposomal composition of the present invention preferably comprises stabilizers, which are selected from the group consisting of α-tocopherol or carbohydrates, in particular glucose, sorbitol, sucrose, maltose, trehalose, lactose, cellubiose, raffinose, maltotriose, or dextran.

It was shown that the liposomes of the present invention and/or the liposomal compositions are capable delivery vehicle to delivery therapeutic and/ diagnostic agents to certain cell types, in particular to cells of the hematopoietic cell lineage for immunization/vaccination strategies used to treat or prevent a disease and, therefore, another aspect of this invention is the use of a liposome of the invention or the liposomal composition of the invention for the production of a medicament for the prevention or therapy of proliferative diseases, infectious diseases, vascular diseases, rheumatoid diseases, inflammatory diseases, immune diseases, in particular autoimmune diseases and allergies.

The liposomes or the liposomal compositions can be administered through a variety of ways including intra-muscular, intravenous, intranasal, intraperitoneal, intradermal, or subcutaneous and intranodal application. The compounds can also be injected directly into the disease site. The liposomes are administered in amounts and intervals, which are commonly used for other vaccination/immunization strategies or in the case of the delivery of drugs at a dose, which is commonly used for the free drug.

In the experiments performed by the present inventors it was shown that the liposomes and liposomal compositions have a superior efficacy in the treatment and/or prevention of tumors and, therefore, in a preferred embodiment the proliferative disease to be treated or prevented is selected from the group consisting of carcinomas of the gastrointestinal or colorectal tract, liver, pancreas, kidney, bladder, prostate, endometrium, ovary, testes, melanoma, dysplastic oral mucosa, invasive oral cancers, small cell and non-small cell lung carcinomas, hormone-dependent breast cancers, independent breast cancers, transitional and squamous cell cancers, neurological malignancies including neuroblastoma, gliomas, astrocytomas, osteosarcomas, soft tissue sarcomas, hemangioamas, endocrinological tumors, hematologic neoplasias including leukemias, lymphomas, and other myeloproliferative and lymphoproliferative diseases, carcinomas in situ, hyperplastic lesions, adenomas, fibromas, histiocytosis, chronic inflammatory proliferative diseases, vascular proliferative diseases and virus-induced proliferative diseases.

In a preferred use of the liposomes and compositions of the invention one or more adjuvants and or cytokines are administered prior, simultaneously or after administration of the liposome or liposomal composition. The term adjuvant is used here as previously defined. Preferred adjuvants are selected from the group of adjuvants consisting of unmethylated DNA comprising CpG dinucleotides (CpG motif), in particular CpG ODN with phosphorothioate (PTO) backbone (CpG PTO ODN) or phosphodiester (PO) backbone (CpG PO ODN); gel-like precipitates of aluminum hydroxide (alum); bacterial products from the outer membrane of Gram-negative bacteria, in particular monophosphoryl lipid A (MPLA), lipopolysaccharides (LPS), muramyl dipeptides and derivatives thereof; synthetic lipopeptide derivatives, in particular Pam₃Cys; lipoarabinomannan; peptidoglycan; zymosan; heat shock proteins (HSP), in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, interleukin- (IL-)2, IL-6, IL-7, IL-18, type I and II, interferons, in particular interferon-gamma, TNF-alpha; oil in water emulsions, in particular MF59 consisting of squalene; Tween 80 and Span 85 (sorbitan-trioleate) and QS-21, a more highly purified derivative of Quil A, non-ionic block polymers, in particular Poloxamer 401, saponins and derivatives thereof, in particular the immunostimulatory fragments from saponins; polyphosphazene; N-(2-Deoxy-2-L-leucylamino-β-D-glucopyranosyl)-N-octadecyldodecanoylamide hydroacetate (BAY R1005), 25-dihydroxyvitamin D3 (calcitriol); DHEA; murametide [MDP(Gln)-OMe]; murapalmitine; polymers of lactic and/or glycolic acid; polymethyl methacrylate; sorbitan trioleate; squalane; stearyl tyrosine; squalene; theramide, synthetic oligopeptides, in particular MHCII-presented peptides. Particular preferred adjuvants, which can be administered prior, during or after administration of the liposomes of the present invention or the liposomal composition are cytokines with adjuvant activity, in particular GM-CSF, interleukin (IL-)2, IL-6, IL-7, IL-18, type I and II, interferons, in particular interferon-gamma, or TNF-alpha.

The following examples are included to demonstrate preferred embodiments of the invention. It should be appreciated by those of skill in the art that the techniques disclosed in the examples that follow represent techniques discovered by the inventors to function well in the practice of the invention, and thus can be considered preferred modes for its practice. However, those of skill in the art should, in light of the present disclosure, appreciate that many changes can be made in the specific embodiments that are disclosed without departing from the spirit and scope of the invention as set out in the appended claims. All references cited are incorporated herein by reference.

### Brief Description of the Figures and Drawings

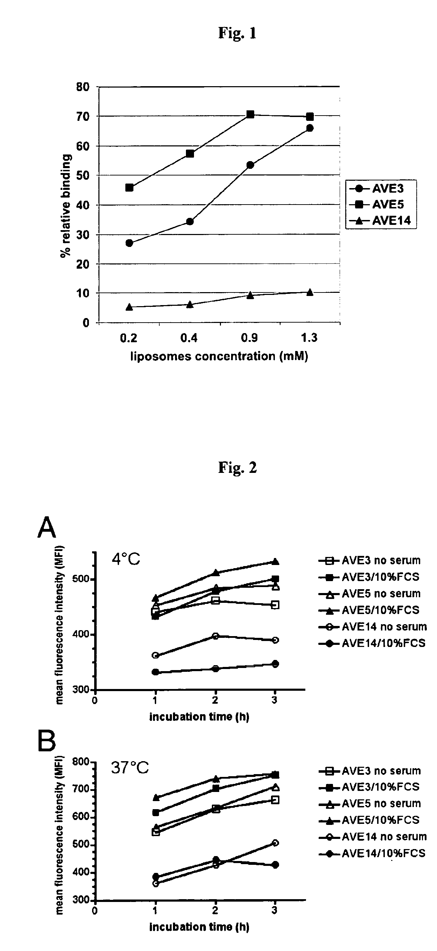

- **Fig. 1:**: **Binding of liposomal formulations to DCs.** Binding of AVE3 and AVE5 but not AVE14 to bone marrow derived murine dendritic cells (bmDC) analyzing varying concentrations of liposomes. Incubations were performed at 8°C and binding was expressed as percentage relative binding to CD 11c-positive cells.
- **Fig. 2:**: **Binding of liposomal formulations to DCs.** Binding of AVE3 and AVE5 but not AVE14 to murine bmDC at 4°C or 37°C in the presence of absence of 10% FCS and dependent on incubation time. Binding is shown as mean fluorescence intensity (MFI).

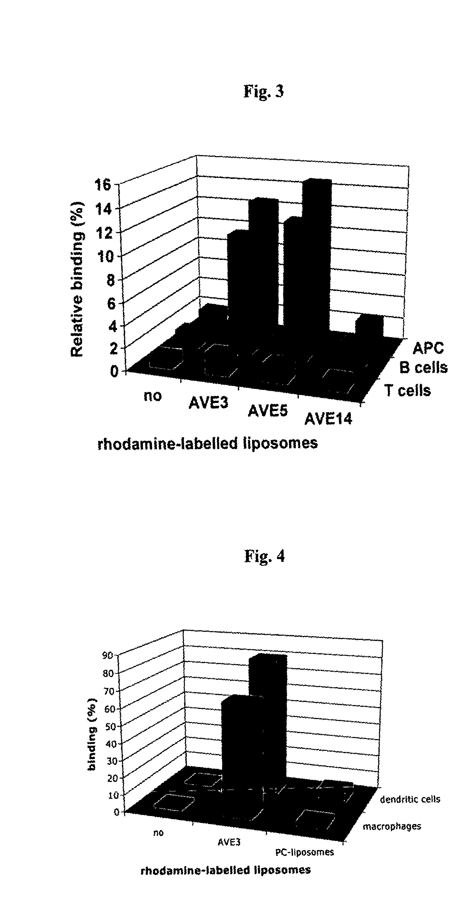

- **Fig. 3:**: **Binding of AVE3 and AVE5 to APCs and B cells.** Binding of AVE3 and AVE5 to murine APCs and B cells but only weakly to T cells.
- **Fig. 4:**: **Binding of AVE3 to human macrophages and dendritic cells.** The binding of AVE3 to human macrophages and dendritic cells derived from peripheral blood mononuclear cells. Binding of rhodamine-labeled AVE3 was analyzed by flow cytometry. PC liposomes (AVE14) were included as negative control.

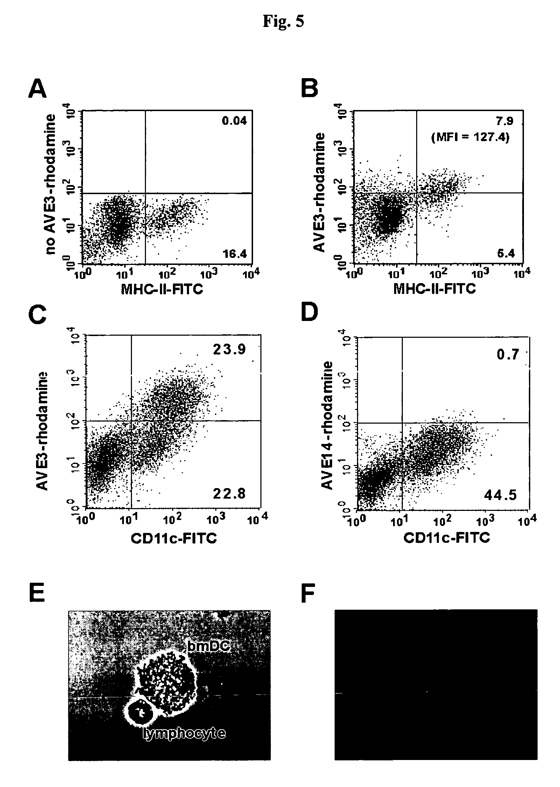

- **Fig. 5:**: **AVE3 bind to Langerhans cells.** Rhodamine-labeled AVE3 bind strongly to Langerhans cells isolated from murine skin (**B**) in comparison to cells incubated without liposome (**A**). Strong binding of AVE3 is also observed for bmDC (**C**) while control liposome AVE14 do not bind to these cells (**D**). Binding and internalization of AVE3 by bmDC as visualized by microscopy. A lymphocyte at the lower left site does not show any binding of AVE3. Phase contrast microscopy (**E**) and fluorescence microscopy (**F**).

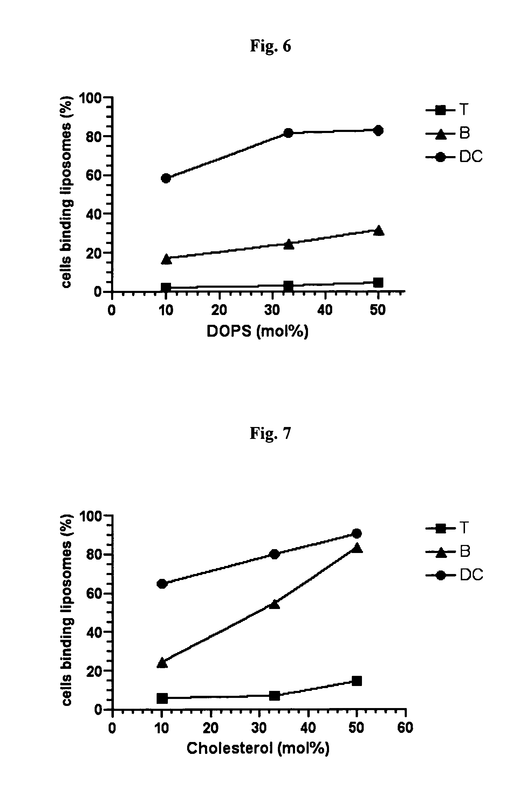

- **Fig. 6:**: **Influence of phosphatidylserine content.** The influence of phosphatidylserine concentration on binding to DCs, B cells, and T cells was assessed. Binding was performed at 4-8°C in medium.
- **Fig. 7:**: **Influence of cholesterol content.** The influence of cholesterol content on liposome binding to DCs, B-, and T-cells was assessed. Binding was performed at 4-8°C in medium.

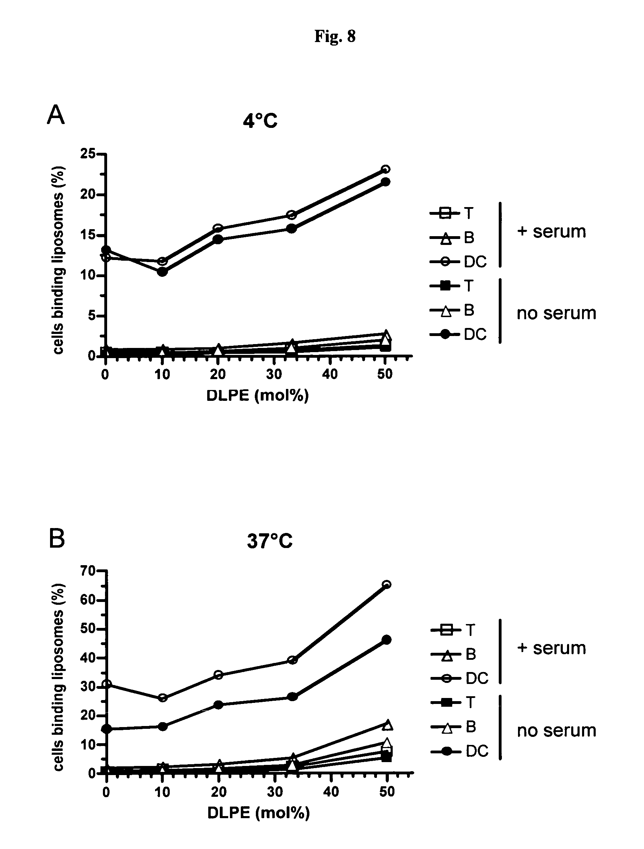

- **Fig. 8:**: **Influence of phosphatidylethanolamine content.** The influence of phosphatidylethanolamine (DLPE) content on liposome binding and internalization to DCs, B-, and T-cells was assessed. Binding was performed at 4°C and 37°C, respectively, with or without 10% serum. Dead cells were excluded by propidium iodide stain (1 µg/ml).

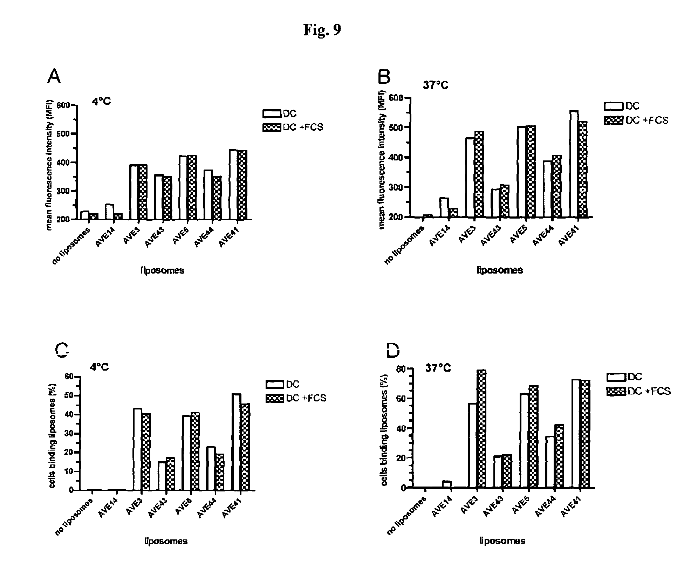

- **Fig. 9:**: **Influence of different lipid compositions on binding to DCs.** Binding was performed at 4°C or 37°C in medium in the presence or absence of 10% FCS was assessed. This experiment demonstrates the beneficial effects of PS or PG in combination with equal molar amounts of PE and cholesterol on binding to DCs. In addition, liposomes consisting of PS, PG and cholesterol, i.e. containing two negatively charged lipids, show similar binding activity as liposomes consisting of one negatively charged phospholipid (PS or PG), PE and cholesterol. (**A**) and (**C**) incubation at 4°C, (**B**) and (**D**) incubation at 37°C, (**A**) and (**B**) mean fluorescence intensity of binding to DCs, (C) and (D) percentage of cells binding liposomes.

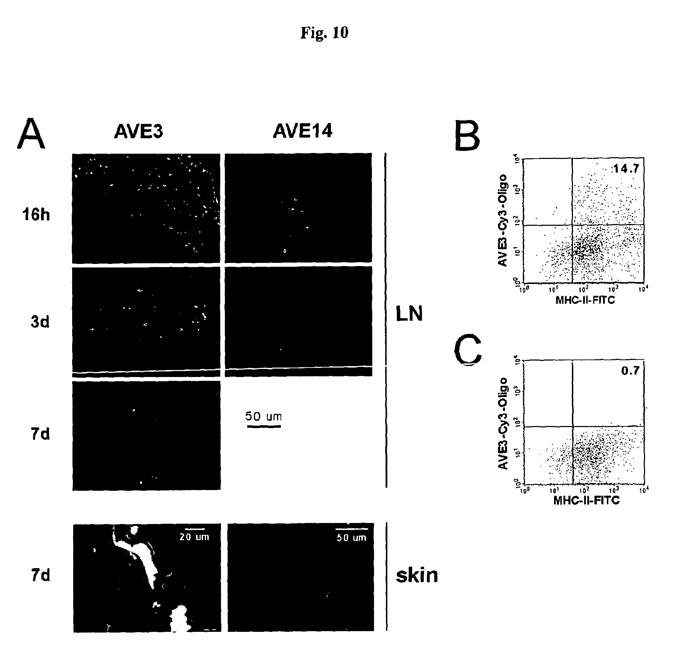

- **Fig. 10:**: **Liposomal deposition.** (**A**) Long-lasting liposomal deposition at the injection site. 7 days after injection a strong accumulation at the injection site (skin) but also in the draining lymph nodes could be detected for rhodamine-labeled AVE3. In contrast, AVE14 did not show accumulation in these organs. Liposomal-entrapped ODNs were taken up by DC (MHC-II positive cells) and were transported to the draining lymph nodes as shown by FACS analysis in mice injected into the hind foot pads (**B**) in comparison to DC from lymph nodes of mice injected id into the flanks (**C**).

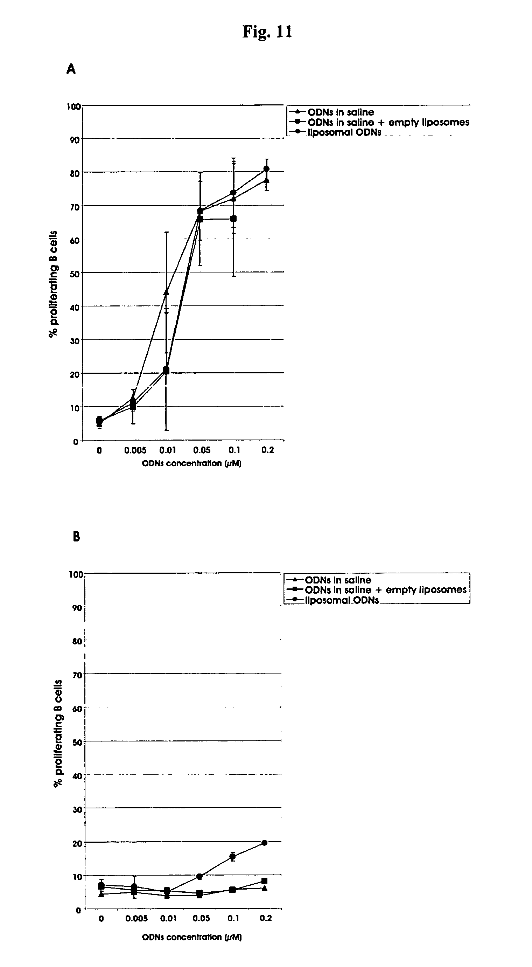

- **Fig. 11:**: **Induction of B cell proliferation.** (**A**) AVE3-encapsulated CpG-PTO oligonucleotides induce B cell proliferation comparable to induction by free CpG-PTO. (**B**) No effects were observed using a non-CpG oligonucleotide demonstrating that B cell proliferation is specifically mediated by CpG oligonucleotides.

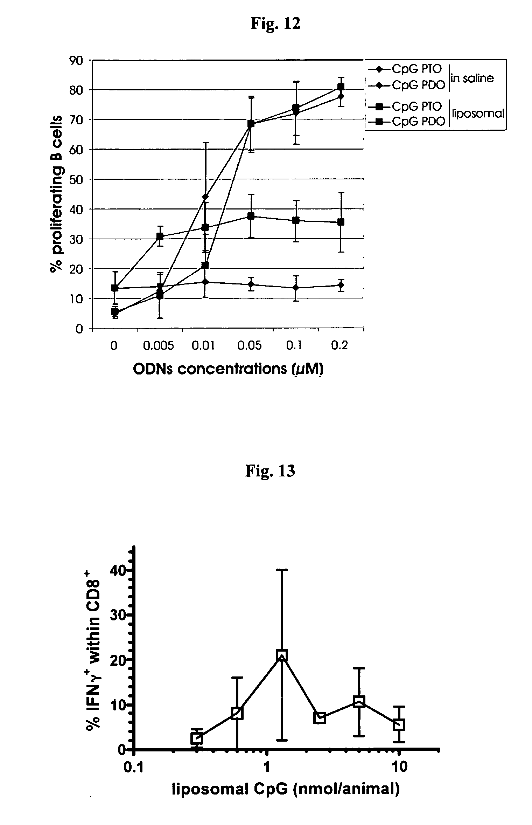

- **Fig. 12:**: **Comparison of the biological activity of CpG-PTO and CpG-PDO.** Cells were labeled with CFSE before being stimulated for 48 hrs with 0 to 0.2 µmol/l end concentration of ODNs in saline or liposomal. (black diamonds) CpG-PTO in saline; (grey diamonds) CpG-PDO in saline; (black squares) liposomal CpG-PTO; (grey squares) liposomal CpG-PDO. Dead cells were excluded by propidium iodide staining.
- **Fig. 13:**: **Titration of liposomal CpG-PTO.** Mice were immunized with 100 µg TRP-2 combined with 0.3 to 10 nmol liposomal CpG-PTO ODNs. Data show per cent of IFNγ-secreting T cells within the CD8+ population after 16 hours restimulation with TRP2 (5 µmol/l). Restimulation with the irrelevant peptide (Ova-peptide SIINFEKL) resulted in IFNγ⁺ cells less than 1.0 %. Dead cells were excluded by propidium iodide staining.

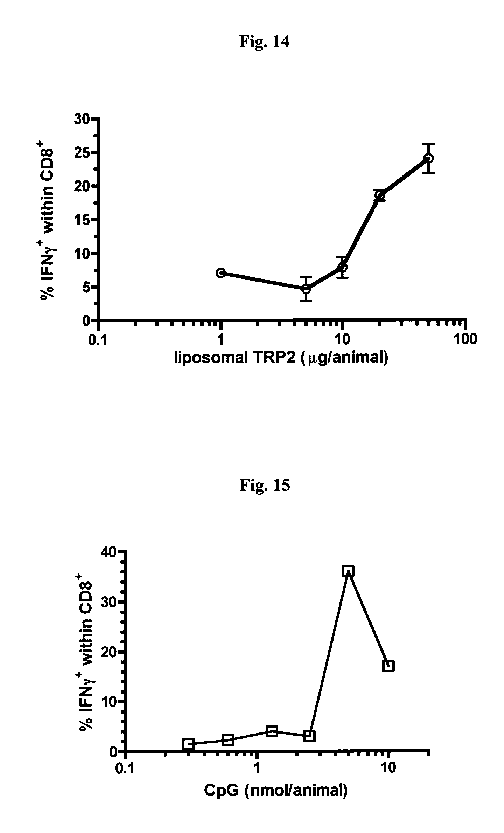

- **Fig. 14:**: **Titration of liposomal TRP-2.** Mice were immunized twice in weekly intervals with AVE3/TRP2 plus 5 nmol CpG-PTO (CpG). Total amount of TRP2 used for immunization was 1 - 50 µg/animal (two animals/group). Data show per cent of IFNγ-secreting T cells within the CD8+ population after 16 hours restimulation with TRP2 (5 µmol/l). Restimulation with the irrelevant peptide (Ova-peptide SIINFEKL) resulted in IFNγ⁺ cells less than 0.5 %.
- **Fig. 15:**: **Titration of CpG-PTO in saline plus 10 µg liposomal TRP-2.** Mice were immunized with 10 µg liposomal TRP-2 combined with 0 to 10 nmol CpG-PTO ODNs. Data show per cent of IFNγ-secreting T cells within the CD8+ population after 16 hours restimulation with TRP2 (5 µmol/l). Restimulation with the irrelevant peptide (Ova-peptide SIINFEKL) resulted in IFNγ⁺ cells less than 1.0%. Dead cells were excluded by propidium iodide staining.

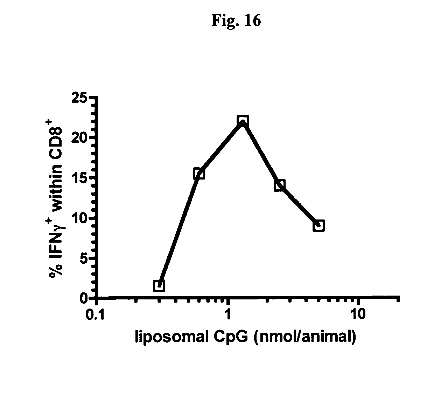

- **Fig. 16:**: **Titration of liposomal CpG-PTO.** Mice were immunized with 10 µg liposomal TRP-2 combined with 0.3 to 5 nmol liposomal CpG-PTO ODNs. Data show per cent of IFNγ-secreting T cells within the CD8+ population after 16 hours restimulation with TRP2 (5 µM). Restimulation with the irrelevant peptide (Ova-peptide SIINFEKL) resulted in IFNγ⁺ cells less than 1.0%. Dead cells were excluded by propidium iodide staining.

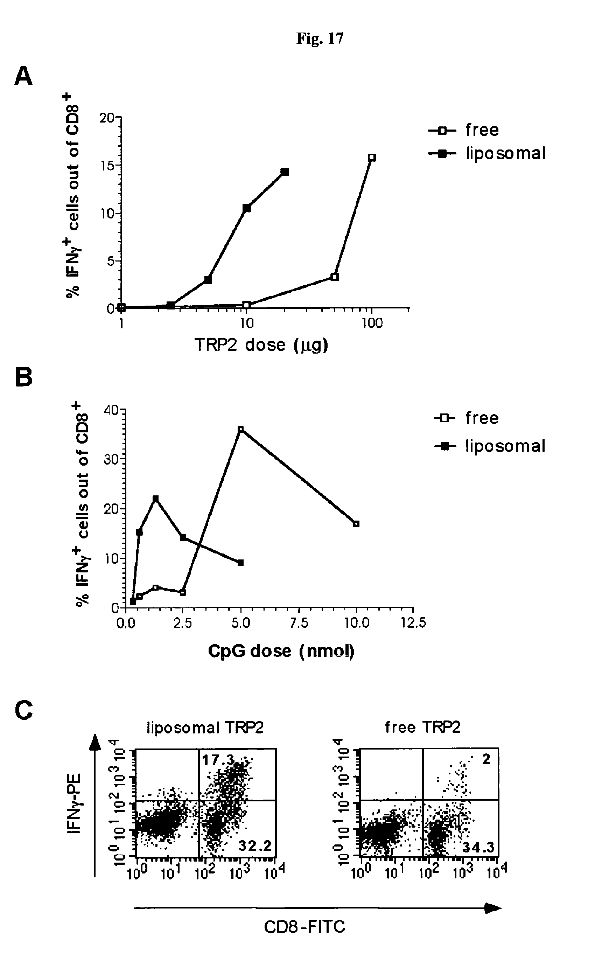

- **Fig. 17:**: **Titration of liposomal TRP-2 and CpG.** The induction of antigen-specific CD8⁺ T cells depending on the TRP-2 and CpG ODN concentration. (**A**), Varying amounts of free (□) or liposome-encapsulated (■) TRP-2 together with 5 nmol free CpG ODN or (**B**), graded doses of free (□) or liposome-encapsulated CpG ODN (■) together with 10 µg liposome-encapsulated TRP-2 or C, 100 µg free (free TRP-2) or 10 µg liposome-encapsulated TRP-2 (liposomal TRP-2) together with 1.3 nmol liposome-encapsulated CpG ODN were injected into the hind footpads of BL6 mice, and 4 days later lymphocytes were isolated from draining LN and cultured in vitro for an additional 6 days in the presence of low dose IL-2 (30U/ml). Lymphocytes were stimulated over night with 5 µmol/l TRP-2 and control peptide SIINFEKL, and an IFNγ capture assay was performed. Percentage of IFNγ positive cells was always below 0.5% with control peptide SIINFEKL. Representative data from one of two similar experiments are shown.

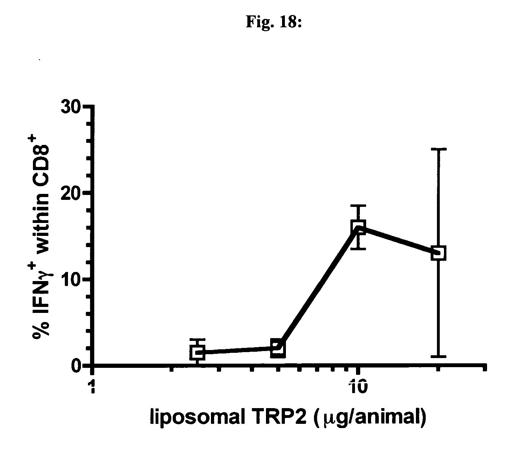

- **Fig. 18:**: **Induction of antigen-specific CD8**^{**+**} **T cells.** Mice were immunized with 2.5 to 20 µg liposomal TRP-2 combined with 1.3 nmol liposomal CpG-PTO ODNs. Data show per cent of IFNγ-secreting T cells within the CD8+ population after 16 hours restimulation with TRP2 (5 µmol/l). Restimulation with the irrelevant peptide (Ova-peptide SIINFEKL) resulted in IFNγ⁺ cells less than 1.0%. Dead cells were excluded by propidium iodide staining.

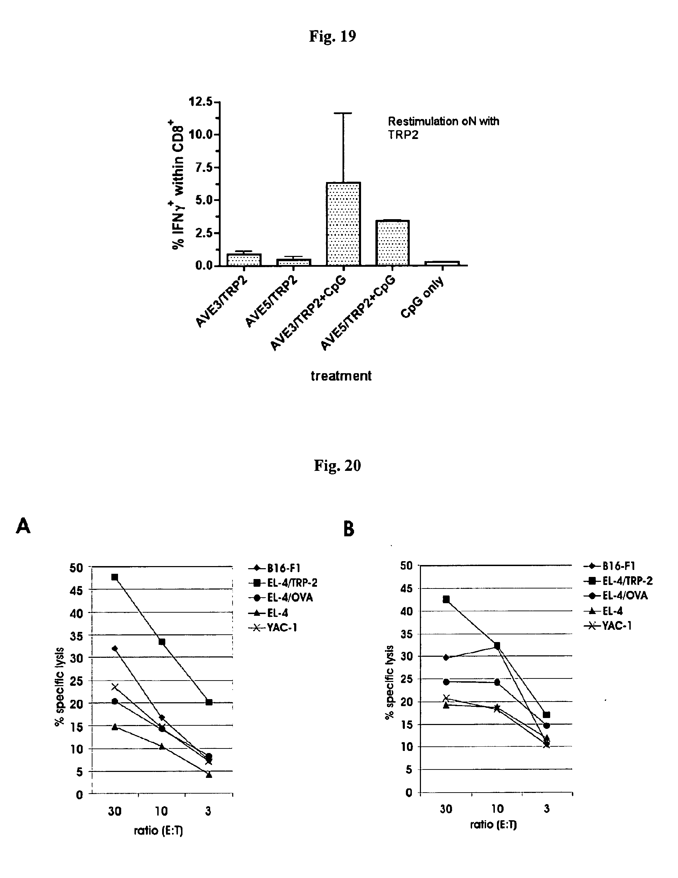

- **Fig. 19:**: **Comparison of induction of IFNγ producing CD8**^{**+**}**.** The comparison of the induction of IFNγ producing CD8⁺ cells after vaccination with 20 µg TRP-2 encapsulated into AVE3 or AVE5 in the presence or absence of free CpG-PTO. Restimulation over night without peptide or with control Ova-peptide (SIINFEKL) resulted in IFNγ⁺ cells below 0.5%. Two mice were used per immunization group.
- Fig. 20:: Comparison of CTL induction after immunization. Mice were immunized either with 100 µg TRP-2 plus 5 nmol CpG-PTO both in saline (A) or with 10 µg TRP-2 plus 1.3 nmol CpG-PTO both liposomal (B).

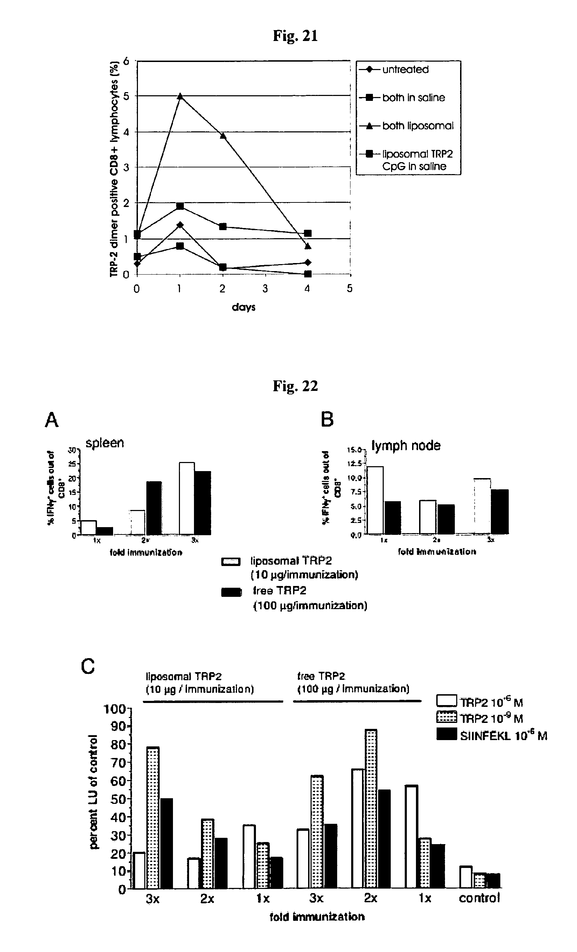

- Fig. 21:: Liposomal vaccination resulted in high frequency of specific T cells in blood. Mice were immunized with either 100 µg TRP-2 plus 5 nmol CpG-PTO in saline (red), 10 µg liposomal TRP-2 plus 5 nmol CpG-PTO in saline (orange), 10 µg liposomal TRP-2 plus 1.3 nmol liposomal CpG-PTO, or left untreated (blue). Given quantities are those injected per animal. Dead cells were excluded by propidium iodide staining.
- **Fig. 22:**: **Vaccination with liposomal-encapsulated TRP-2 plus CpG-PTO.** The vaccination with liposomal-encapsulated TRP-2 plus CpG-PTO generates high avidity T cells. BL6 mice were vaccinated with 10 µg liposomal or 100 µg free TRP-2 plus 5 nmol free CpG ODN at weekly intervals up to three times. Lymphocytes were isolated from spleen (A) and lymph node (LN) (B) one week after last immunization and an IFNγ capture assay was performed as described. C, Avidity of T cells after vaccination with liposomal and free TRP-2 plus CpG ODN. One week after the last immunization splenocytes were nylon-wool enriched and cultured for 5 days in vitro in the presence of high dose (10⁻⁶ mol/l) or low dose TRP-2 (10⁻⁹ mol/l) or control peptide SIINFEKL (10⁻⁶ mol/l) with low dose IL2 (30 U/ml). Proliferation was estimated by a luminescence assay-based detection system measuring ATP content. Proliferation of splenocytes in the presence of anti-TCR plus anti-CD28 mAb was set to 100%. Representative data from one of two similar experiments are shown.

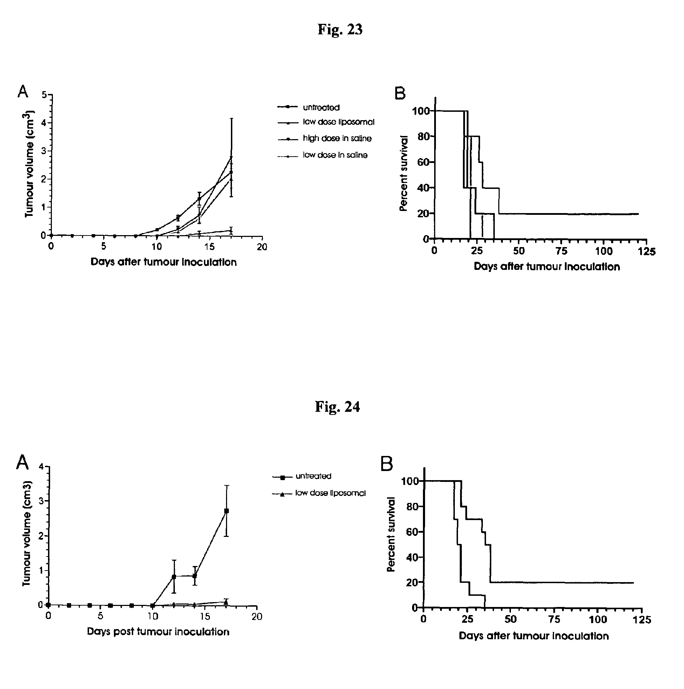

- **Fig. 23:**: (**A**) Low doses of liposomal vaccination prevent tumor growth and (**B**) prolong survival in a prophylactic setting. Mice were immunized twice with 100 µg TRP-2 plus 5 nmol CpG-PTO in saline (dark blue; n = 4), 10 µg TRP-2 plus 1.3 nmol CpG-PTO in saline (light blue; n = 4), 10 µg liposomal TRP-2 plus 1.3 nmol liposomal CpG-PTO (green; n = 4), or left untreated (red; n = 4). Mice were subcutaneously inoculated with 2 x 10⁵ B16 melanoma cells. Sizes of the tumor were measured from day 9.
- **Fig. 24:**: (**A**) Tumor rejection after liposomal vaccination and (**B**) prolonged survival in a therapeutic setting. Mice were subcutaneously inoculated with 1 x 10⁵ B16 melanoma cells, before being immunized twice with 10 µg liposomal TRP-2 plus 1.3 nmol liposomal CpG-PTO green; n=10), or left untreated (red; n=10). Sizes of the tumor were measured from day 10. P< 0.0012: low liposomal dose versus untreated).

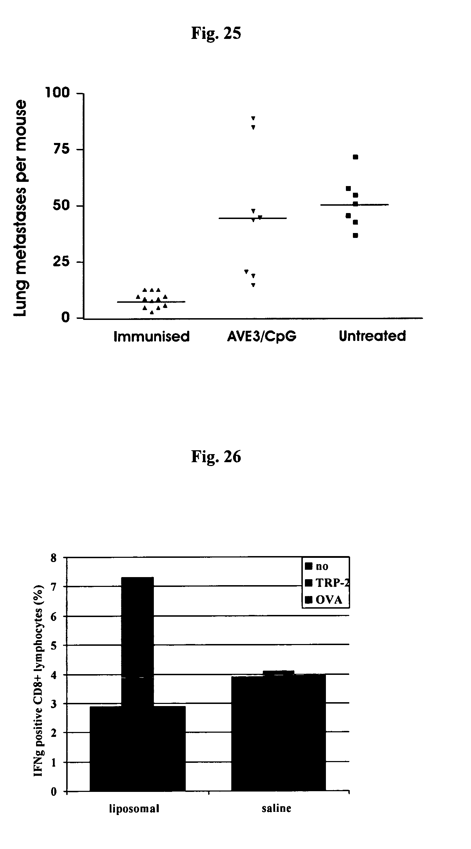

- **Fig. 25:**: Number of the B 16 melanoma metastatic colonies in the lungs after immunization in a prophylactic setting. Mice were immunized twice with 10 µg liposomal TRP-2 plus 1.3 nmol liposomal CpG-PTO (immunized; n = 12), 1.3 nmol liposomal CpG-PTO (AVE3/CpG; n = 8), or left untreated (untreated; n = 7). One week after the last immunization, mice received one lethal injection of B16 melanoma cells via tail vein injection. Colonies on the lungs were counted. ― , average metastatic colonies in each group. Significant (Student's t test) difference was found: immunized versus untreated, p < 0.0001; immunized versus AVE3/CpG, p < 0.0003.
- **Fig. 26:**: **Liposomal encapsulation of CpG-PDO improved adjuvant efficiency.** Mice were immunized with 10 µg liposomal TRP-2 combined with either 5 nmol CpG in saline (saline) or 1.3 nmol liposomal CpG-PDO (liposomal). (no) untreated; (TRP-2) 16 hrs re-stimulation in vitro with 5 µmol/l TRP-2 peptide; (OVA) 16 hrs re-stimulation in vitro with 5 µmol/l OVA peptide. Dead cells were excluded by propidium iodide staining.

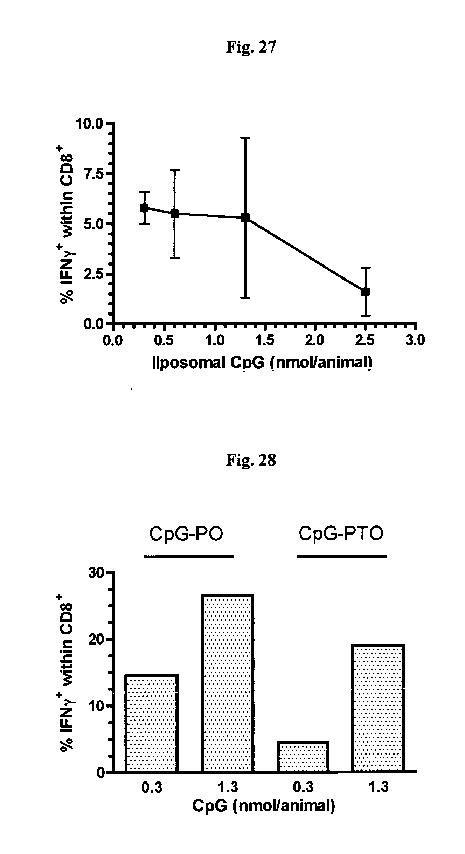

- **Fig. 27:**: **Titration of liposomal CpG-PDO.** Mice were immunized with 10 µg liposomal TRP-2 combined with 0.3 to 2.5 nmol liposomal CpG-PDO. (no) untreated; (TRP-2) 16 hrs re-stimulation in vitro with 5 µmol/l TRP-2 peptide; (OVA) 16 hrs restimulation in vitro with 5 µmol/l OVA peptide. Dead cells were excluded by propidium iodide staining.
- **Fig. 28:**: **Comparison of liposomal CpG-PDO with liposomal CpG-PTO.** Mice were immunized with 10 µg liposomal TRP-2 combined with 0.3 or 1.3 nmol liposomal CpG-PDO or liposomal CpG-PTO. (no) untreated; (TRP-2) 16 hrs re-stimulation in vitro with 5 µmol/l TRP-2 peptide; (OVA) 16 hrs re-stimulation in vitro with 5 µmol/l OVA peptide. Dead cells were excluded by propidium iodide staining.

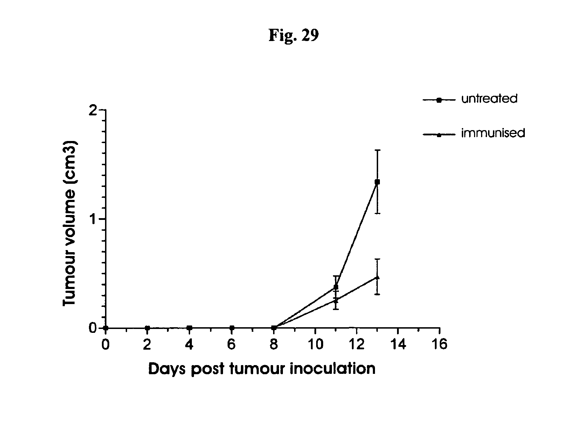

- **Fig. 29:**: **Low doses of liposomal vaccination reduce tumor growth.** Mice were immunized twice 10 µg liposomal TRP-2 plus 0.3 nmol liposomal CpG-PDO (13 nmol/kg) (green; n = 4), or left untreated (red; n = 4). Mice were subcutaneously inoculated with 2 x 10⁵ B16 melanoma cells. Sizes of the tumor were measured from day 8.

### Examples

### Example 1: Binding of AVE3 and AVE5 to antigen-presenting cells

Various liposomal formulations were analyzed for binding to various types of antigen-presenting cells: AVE3 (cholesterol, DLPE, DOPS at a molar ratio of 1:1:1), AVE5 (cholesterol, DLPE, DOPG at a molar ratio of 1:1:1), or AVE14 (cholesterol, DLPE, EPC at a molar ratio of 1:1:1). All lipids were purchased from Avanti Polar Lipids (USA), Calbiochem (USA) or Lipoid GmbH (Germany) and were used without further purification. Liposomes were prepared from dried lipid films by hydration. For this purpose lipids were dissolved in chloroform or chloroform/methanol (1:1) and mixed at the indicated ratios. For binding studies 0.3 mol% rhodamine-labeled DPPE was added. Lipids were dried using a rotary evaporator and residual solvent was removed under high vacuum. Lipid films were then hydrated with 10 mmol/l Hepes pH 7.4 to a final lipid concentration of 10 µmol/ml. Liposomes were extruded 21-times through 50 nm membranes. All liposomes prepared had an average size of 80 - 110 nm. Average zeta potentials were -67 mV for AVE3, -43 mV for AVE5, and -12 mV for AVE14.

Murine dendritic cells were generated from bone marrow cells collected from femurs of naive C57BL/6 mice and cultured for six days in the presence of mGM-CSF. These bone marrow-derived dendritic cells (bmDCs) were incubated for 30 minutes at 8°C with 0.2 to 1.3 mmol/l rhodamine-labeled liposome formulations in serum-free medium or medium containing 10% FCS. The cells were then washed twice with PBS and DCs were counterstained with monoclonal antibodies against CD11c-FITC and analyzed by flow cytometry. The results (Fig. 1) showed that negatively charged PS- or PG-containing liposomes (AVE3 and AVE5) but not AVE14 liposomes bind strongly to murine bmDCs. In these experiments an increased binding was observed as determined by analysis of mean fluorescence intensity (MFI) for liposomes incubated in the presence of 10% FCS (Fig. 2). In addition, fluorescence intensity was improved by incubating cells at 37°C (Fig. 2).

In a further study binding to murine spleen-derived antigen-presenting cells (APC), T cells, or B cells was analyzed. APCs were identified by staining with anti-MHC II antibodies, T cells by staining with anti-CD3 antibodies, and B cells by staining with anti-CD45R/B220.

This study demonstrated strong binding of AVE3 or AVE5 to APC and B cells while only marginal binding was observed for AVE14 (Fig. 4).

Strong binding of AVE3 was also observed with human macrophages or dendritic cells generated by cultivating peripheral blood mononuclear cells for one week with IL-4 and GM-CSF (Fig. 4). In contrast, PC-liposomes consisting of 35 mol% cholesterol, 32.1 mol% POPC, 14.7 mol% DLPE, 18.2 mol% milk SM did show only very week binding to these cells.

It was further analyzed whether liposomes used for immunization bind to Langerhans cells (LC) of the skin, as these cells are the first one capturing antigen upon id vaccination. Primary LC were isolated from murine skin and incubated with liposomes as described. Approximately 60 % of MHC class II⁺ LC bound AVE3 liposomes (Fig. 5A, B). In addition, as described before murine CD11c⁺ bmDC also bound AVE3-liposomes. Moreover, within the CD11c⁺ population of DC about 54 % bind AVE3-liposomes, but did not bind the control liposomes AVE14 (Fig. 5C, D). In parallel to FACS analysis, bmDC binding of AVE liposomes was also analyzed by fluorescence microscopy (Fig. 5). A strong intracellular fluorescence was observed for bmDCs indicating that liposomes become internalized after binding to DCs.

These experiments demonstrate that AVE3 but also AVE5 possess strong binding activity for various antigen-presenting cells of the immune system. This binding is also observed at 37°C in the presence of serum proteins demonstrating that these liposomes exhibit binding activity for these cells under physiological conditions.

### Example 2: Influence of phosphatidylserine, cholesterol, and phosphatidylethanolamine concentration on binding to antigen-presenting cells.

In a first experiment the influence of varying phosphatidylserine (DOPS) concentrations on binding to dendritic cells, B cells and T cells isolated from murine spleens was analyzed. In order to keep the concentrations of the other lipids constant the following liposomes were produced: AVE31 (25 mol%, cholesterol, 25 mol% DLPE, 33.3 mol% DOPS, 16.7 mol% POPC), AVE31-10PS (25 mol%, cholesterol, 25 mol% DLPE, 10 mol% DOPS, 40 mol% POPC), AVE31-50PS (25 mol%, cholesterol, 25 mol% DLPE, 50 mol% DOPS) adding 0.3 mol% rhodamine-labeled PE for detection. Murine spleen cells were incubated with liposomes at 4 - 8°C. DCs were identified by counter-staining with anti-CD11c antibody, T cells with anti-CD3 antibody, and B cells with anti-CD45R antibody. Strong binding was observed to DCs but also to B cells for all liposomes (5 x 10⁵ spleen cells incubated with 200 nmol lipid), while only weak binding was observed with T cells. Binding depended on the PS concentrations with strong binding to DCs observed at 33.3 and 50 mol% (approximately 80-90% of the CD11c-positive cells binding) while at 10 mol% PS binding was reduced to approximately 65% (Fig. 6). Similar binding properties were observed in the presence of 10% FCS in the medium. These experiments clearly demonstrated that strongest binding to DCs and other antigen-presenting cells such as B cells is observed at a PS concentration of 20-50 mol%. This binding behavior was also observed in the presence of 10% FCS.

In a second experiment the influence of varying cholesterol concentrations on binding to dendritic cells, B cells and T cells was analyzed. The following liposomes were produced: AVE32 (33.3 mol%, cholesterol, 25 mol% DLPE, 25 mol% DOPS, 16.7 mol% POPC), AVE32-10Chol (10 mol%, cholesterol, 25 mol% DLPE, 25 mol% DOPS, 40 mol% POPC), AVE32-50Chol (50 mol%, cholesterol, 25 mol% DLPE, 25 mol% DOPS) adding 0.3 mol% rhodamine-labeled PE for detection. Murine spleen cells were incubated with liposomes at 4 - 8°C. DCs, B cells and T cells were identified by antibody staining as described above. Again, strong binding was observed to DCs and B cells. Binding depended on the cholesterol concentration with the strongest binding observed at 50 mol% cholesterol (Fig. 7).

In a third experiment the influence of varying phosphatidylethanolamine concentrations on binding to dendritic cells, B cells and T cells was analyzed. The following liposomes were produced: AVE33-0PE (25 mol%, cholesterol, 25 mol% DOPS, 50 mol% POPC), AVE33-10PE (25 mol%, cholesterol, 10 mol% DLPE, 25 mol% DOPS, 40 mol% POPC), AVE33-20PE (25 mol%, cholesterol, 20 mol% DLPE, 25 mol% DOPS, 30 mol% POPC), AVE33-33PE (25 mol%, cholesterol, 33.3 mol% DLPE, 25 mol% DOPS, 16.7 mol% POPC), AVE33-50PE (25 mol%, cholesterol, 50 mol% DLPE, 25 mol% DOPS), adding 0.3 mol% rhodamine-labeled PE for detection. Murine spleen cells were incubated with liposomes at 4 - 8°C. DCs, B cells and T cells were identified by antibody staining as described above. An increase in the PE concentration resulted in an increase in binding of the liposomes to DCs and to some extend also toB cells and T cells (Fig. 8). This finding demonstrates that increasing the PE concentration improves binding and uptake of liposomes.

In a final experiment liposomes consisting of varying compositions of PS, PG, PE, Chol, and PC for binding to DC were analyzed. The following liposomes were tested: AVE3 (DOPS, DLPE, Chol at a molar ratio of 1:1:1), AVE5 (DOPG, DLPE, Chol at a molar ratio of 1:1:1), AVE 14 (EPC, DLPE, Chol at a molar ratio of 1:1:1), AVE41 (DOPS, DOPG, Chol at a molar ratio of 1:1:1), AVE43 (DOPS, DPPC, Chol at a molar ratio of 1:1:1), and AVE44 (DOPG, DPPC, Chol at a molar ratio of 1:1:1), adding 0.3 mol% rhodamine-labeled PE for detection. The composistions are summarized in Table 1 below:

**Table 1**

| | **PE** | **PS** | **PG** | **Chol** | **PC** |
|---|---|---|---|---|---|
| **AVE14** | 33.3 | - | - | 33.3 | 33.3 |
| **AVE3** | 33.3 | 33.3 | - | 33.3 | - |
| **AVE43** | - | 33.3 | - | 33.3 | 33.3 |
| **AVE5** | 33.3 | - | 33.3 | 33.3 | - |
| **AVE44** | - | - | 33.3 | 33.3 | 33.3 |
| **AVE41** | - | 33.3 | 33.3 | 33.3 | - |

Murine spleen cells were incubated with liposomes either at 4°C or at 37°C in medium in the presence or absence of 10% FCS. DCs were identified by antibody staining as described above. As observed before (example 1) AVE3 and AVE5 bound strongly to dendritic cells, while AVE14 showed only very weak binding. The substitution of PE by PC in AVE3 (= AVE43) or AVE5 (= AVE44) resulted in drastic reduction of binding to DCs as revealed by the mean fluorescence intensity as well as the percentage of cells binding to DCs (Fig. 9). This difference was strongest when analyzing liposomes at 37°C indicating that PE has some effects on uptake of liposomes by DCs. This finding clearly demonstrates that the presence of PE in AVE3 or AVE5 has beneficial effects on binding to and uptake by dendritic cells. It also shows that PE in the absence of PS or PG (= AVE14) is not sufficient for binding of liposomes to DCs. Surprisingly, it was found however that liposomes consisting of equal molar amounts of PS, PG and cholesterol (AVE41) showed similar binding activity as AVE3 or AVE5 (Fig. 9). This finding indicates that liposomes consisting of two negatively charged phospholipids (PS and PG) in combination with cholesterol can substitute liposomes consisting of cholesterol, PE and either PS or PG.

### Example 3: Long-lasting liposome deposition at the site of injection and the draining lymph nodes

In order to evaluate the availability and the stability of different liposomal formulations in vivo rhodamine-labeled AVE3 or AVE14 liposomes (see example 1) were injected id into the flank or into the hind footpad of C57BL/6 mice. At different time points skin from the injection site as well as draining lymph nodes were embedded and cryosections were prepared (Fig. 10A). A strong fluorescence signal was observed within the skin up to 7 days after AVE3 injection, whereas no significant fluorescence could be detected in the case of AVE14 liposomes (Fig. 10A). Furthermore, in the draining lymph nodes, fluorescence could be detected up to 7 days after injection only in the case of AVE3, whereas only a weak fluorescence of the neutral liposomes (AVE14) could be detected 16 hrs after injection. These findings indicate that AVE3 result in long-lasting deposition at the injection site and the draining lymph nodes.

### Example 4: Uptake of encapsulated compounds by dendritic cells and localization in the draining lymph nodes

In order to further demonstrate that liposome-entrapped molecules are also taken up by DCs/LC and transported to the lymphoid system, Cy3-labeled non-CpG oligonucleotides (ODN) were encapsulated in AVE3 liposomes (see example 6). A non-CpG ODN was encapsulated to exclude CpG ODNs-mediated activation effects on DCs/LC and thereby an effect on their migration. 24 hrs after id injection into the flanks or into the hind footpads, lymph nodes (LN) were collected and a single cell suspension was prepared and analyzed by FACS. A significant number of DCs (14.7%) from mice injected into the hind footpads had taken up liposome-entrapped Cy3-ODNs, but only a few DCs from mice injected id into the flanks (Fig. 10B, C).

### Example 5: Encapsulation of antigenic peptides into AVE3 and AVE5

The antigenic peptide TRP-2 (SVYDFFVWL) was encapsulated into AVE3 or AVE5 (see example 1 for composition). The lipids were mixed with 29.4 mg TRP-2 at a molar ratio of 1:20 and filled into a 50 ml Duran glass bottle. 50 g Hepes buffer (10 mmol/l, pH 7.4) were added and the mixture was stirred with an Ultra-Turrax T8 dispersing instrument (IKA Werke, Staufen, Germany) for 30 minutes at approx. 25000 rpm in a 55 °C water bath. The Ultra-Turrax was equipped with a S8N-8G dispersing element. Meanwhile the bottle was vortexed several times to avoid precipitations in the comers of the bottle. After obtaining a homogeneous suspension, the evaporated water was replaced and the preparation was transferred in an Emulsiflex C-5 Homogenizer (Avestin Inc., Ottawa, Canada). The following homogenization was performed for 30 minutes between 50.000 and 150.000 kPa in front of the homogenization nozzle and the homogenizer pressure was 300 kPa. At the end of this step the filter unit was inserted and the liposomal dispersion was filtered for 5 minutes through polycarbonate membranes with 100 nm pores before being sterile-filtered. After a one-day storage, the particle size was measured by photon correlation spectroscopy and the amount of TRP-2 in the liposomes by HPLC analysis. Size of liposomes was between 130 to 180 nm. Encapsulation efficiency was in the range of 230 - 300 µg/ml (39-51%).

A side-by-side comparison of various AVE3-based formulations containing 10, 20 or 40 mol% phosphatidylserine demonstrated that increased concentrations of PS improve encapsulation efficiency. The following formulations were tested: AVE3-10PS (33.3 mol%, cholesterol, 33.3 mol% DLPE, 10 mol% DOPS, 23.3 mol% POPC), AVE3-20PS (33.3 mol%, cholesterol, 33.3 mol% DLPE, 20 mol% DOPS, 13.3 mol% POPC), AVE3-40PS (33.3 mol%, cholesterol, 26.7 mol% DLPE, 40 mol% DOPS), and AVE14 (see example 1). Encapsulation efficiency was 28% for AVE3-10PS, 34% for AVE3-20PS, 43% for AVE3-40PS, and 13% for AVE14. Liposome sizes ranged between 100 to 120 nm. This finding demonstrates that PS has an Influence on encapsulation of an antigenic peptide and that higher PS concentrations improve encapsulation.

### Example 6: Encapsulation of oligonucleotides into AVE3

The lipids (40 µmol/ml) (see example 1 for lipid composition of AVE3) dissolved in chloroform or chloroform:methanol (1:1) were dried in a rotary evaporator at 30 mbar and 34 °C for 15 minutes. The resulting lipid film was further dried in a vacuum chamber at 10 mbar. Afterwards, the dried film was hydrated in a rotary evaporator with a few glass beads in 1 ml isotonic Hepes buffer (10 mmol/l; pH 7.4) containing 10 mg/ml oligonucleotides CpG-1826 or non-CpG-1982 (CpG-1826, 5'-TCCATGACGTTCCTGACGTT-3' as phosphorothioate (PTO) and phosphodiester (PDO); non-CpG-1982-PTO, 5'-TCCAGGACTTCTCTCAGGT-3'). The dispersion was then extruded 21 times through polycarbonate membranes having pores with a size of 50 nm. In order to remove the free oligonucleotides, the liposomal suspension was subjected to size exclusion chromatography on a sepharose CL-4B column (Pharmacia, Sweden). The collected liposome fractions were then concentrated by ultrafiltration using Vivaspin concentrators with a cutoff of 30000 MW (Vivascience, Germany). Finally the vesicles were filtered through 200 nm sterile filter membranes. The hydrodynamic diameter was measured using a Zetasizer 3000 HS (Malvern, Herrenberg, Germany). Encapsulated oligonucleotides were determined by ion-exchange HPLC. Size of liposomes was between 100 to 125 nm. Encapsulation efficiency was in the range of 4 to 7%. No differences were observed for phosphorothioate or phosphodiester oligonucleotides demonstrating that phosphorothioate as well as phosphodiester oligonucleotides can both be encapsulated into AVE3 liposomes.

### Example 7: Encapsulation of Pam₃Cys and antigenic peptide into AVE3

Lipids (see example 1) as well as Pam₃Cys (2.5 mol%) dissolved in chloroform or chloroform:methanol (1:1) and the antigenic peptide dissolved in DMSO were dried in a rotary evaporator at 10 mbar and 34 °C for 60 minutes. The resulting lipid film was resolved in chloroform and dried again as described before. This step was repeated till a smooth and homogeneous film was obtained, followed by the removal of residual solvent in a vacuum chamber at 10 mbar. The dried film was then hydrated in a rotary evaporator with a few glass beads in 1 ml isotonic Hepes buffer (10 mmol/l; pH 7.4). The obtained dispersion was extruded 21 times through polycarbonate membranes having pores with a size of 100 nm. At the end of this procedure the formulation was sterile-filtered.

### Example 8: Encapsulation of monophosphoryl-lipid A (MPLA) and antigenic peptide into AVE3

Lipids (see example 1) as well as MPLA (5 or 10% (w/w)) dissolved in chloroform or chloroform:methanol (1:1) and the antigenic peptide dissolved in DMSO were dried in a rotary evaporator at 10 mbar and 34 °C for 60 minutes. The resulting lipid film was resolved in chloroform and dried again as described before. This step was repeated till a smooth and homogeneous film was obtained, followed by removal of residual solvent in a vacuum chamber at 10 mbar. The dried film was then hydrated in a rotary evaporator with a few glass beads in 1 ml isotonic Hepes buffer (10 mmol/l; pH 7.4). The obtained dispersion was extruded 21 times through polycarbonate membranes having pores with a size of 100 nm. At the end of this procedure the formulation was sterile-filtered.

### Example 9: AVE3-encapsulated CpG-PTO oligonucleotides induce B cell proliferation

CpG oligonucleotides have been described to induce B cell proliferation. The effects of free or encapsulated CpG phosphorothioate oligonucleotides (CpG-PTO) (see example 6) on proliferation of murine B cells (Fig. 11A) were compared. It was found that AVE3-encapsulated CpG showed the same activity as unencapsulated CpG-PTO (Fig. 10). Induction of B cell proliferation was dependent on the concentration of applied ODN with strong B cell proliferation observed between 50 to 200 nmol/l encapsulated ODN. Similar values were obtained for CpG-ODN encapsulated at different concentrations (between 4.6 µg/µmol lipid to 93.5µg/µmol lipid). No induction of B cell proliferation was observed using free or encapsulated non-CpG-ODN (Fig. 11B). This finding demonstrates that AVE3-encapsulated CpG-ODNs are biologically active.

### Example 10: AVE3 protects CpG-PDO oligonucleotides from degradation

CpG phosphorothioate (CpG-PTO) or CpG phosphodiester (CpG-PDO) oligonucleotides (see example 6) were analyzed for the induction of B cell proliferation in free or AVE3-encapsulated form using ODN concentrations between 5 to 200 nmol/l. Both free and encapsulated CpG-PTO resulted in a concentration dependent induction of B cell proliferation (Fig. 12). Induction was also observed for AVE3-encapsulated CpG-PDO. At low concentrations (5 to 10 nmol/l) a stronger induction was observed for encapsulated CpG-PDO than with encapsulated CpG-PTO. At higher concentrations (50 to 200 nmol/l) induction was higher for encapsulated CpG-PTO. Most interestingly, free CpG-PDO did not induce any proliferation at these concentrations most likely due to rapid degradation. Thus AVE3 liposomes are able to protect sensitive compounds, such as phosphodiester oligonucleotides, from degradation and allow delivery of molecules in an active form.

### Example 11: Induction of a specific and high-avidity T cell immune response after liposomal vaccination

The effects of various AVE3-based formulations on the generation of an immune response in mice using a TRP-2 peptide (SVYDFFVWL) as antigenic model peptide were analyzed. This peptide is presented by human HLA-A*0201 but also by murine MHC class I molecule H-2K^{b} and the syngenic B16 melanoma cell line. For this purpose mice (C57BL/6) were immunized by a single injection into the hind footpad. After 4 days mice were sacrificed, the draining lymph nodes were removed and cells from the lymph nodes were cultured for 6-7 days in the presence of IL-2. Cells were then stimulated with the antigenic peptide (TRP-2) or an irrelevant peptide (OVA peptide; SIINFEKL). After 16 hours CD8-positive cells were analyzed in a cytokine secretion assay for the production of IFNγ. In all experiments dead cells were excluded by staining with propidium iodide.

In initial experiments it was found that free TRP-2 resulted in a strong induction of IFNγ-producing CD8⁺ cells at a concentration of 50 to 100 µg per animal. Unencapsulated CpG gave a strong immune response at concentrations of 2.5 to 5 nmol per animal.

The effects of varying concentrations of AVE3-encapsulated CpG-PTO in the presence of 100 µg unencapsulated TRP-2 (Fig. 13) were analyzed. This experiment showed a strong induction of IFNγ-producing CD8⁺ cells around a concentration of 1.3 nmol liposomal CpG-ODNs. Higher concentrations did not improve the immune response indicating that low amounts of encapsulated CpG-PTO are sufficient for the induction of a T cell response.

Titration of AVE3-encapsulated TRP-2 in the presence of 5 nmol free CpG-PTO showed a strong induction of IFNγ-producing CD8⁺ cells at 10 to 20 µg peptide per animal (Fig. 14). Titration of free CpG-PTO in the presence of 10 µg AVE3-encapsulated TRP-2 showed a strong induction of IFNγ-producing CD8⁺ cells at 5 to 10 nmol CpG-PTO per animal (Fig. 15).

In further experiments the effects of liposomal TRP-2 and liposomal CpG-PTO (Fig. 16) were compared. Using 10 µg per animal of AVE3-encapsulated TRP-2 a strong induction of IFNγ-producing CD8⁺ cells between 0.6 to 2.5 nmol AVE3-encapsulated CpG-PTO per animal was found. Applying 1.3 nmol AVE3-encapsulated CpG-PTO per animal a strong induction of AVE3-encapsulated CpG-PTO at 10 to 20 µg AVE3-encapsulated TRP-2 (Fig. 17) was observed. Compared to the usage of unencapsulated TRP-2 and CpG-PTO the same induction of IFNγ-producing CD8⁺ cells could only observed using 100 µg free TRP-2 and 5 nmol CpG-PTO. Thus, the encapsulation of these compounds into AVE3 allows for a 4- to 10-fold reduction of the amounts of antigenic peptide and CpG-PTO (Fig. 18).

A comparison of TRP-2 encapsulated into AVE3 or AVE5 showed that at an applied peptide concentration of 10 µg per animal a much strong induction of IFNγ-producing CD8⁺ cells was observed after vaccination with AVE3 than with AVE5, which required coapplication of CpG-PTO as adjuvant. CpG-PTO alone or liposomes without adjuvant did not lead to significant levels of IFNγ-producing CD8⁺ cells (Fig. 19).

In another set of experiments antigen-specificity of cytotoxic T lymphocytes after liposomal vaccination (Fig. 20) was analyzed. Mice were immunized with AVE3-encapsulated or free TRP-2 and CpG-PTO as described above. Cells prepared from lymph nodes were then tested for cytotoxicity using as targets the mouse melanoma cell line B16.F1 know to present TRP-2 in the context of H-2K^{b}, TRP-2 pulsed EL4 cells, OVA peptide-pulsed EL4 cells, unpulsed EL4 cells, and YAC-1 cells to exclude a potential NK cell activity. A specific CTL-mediated lysis of TRP-2 presenting cells (B16.F1 and TRP-2 pulsed EL4) at varying effector to target cell ratios after immunization with 10 µg liposomal TRP-2 and 1.3 nmol liposomal CpG-PTO was demonstrated. No killing of YAC-1 cells was observed indicating that CTLs display an antigen-specific but not a NK-like cytotoxic activity. As described above 10-fold higher amounts of free TRP-2 and 4-fold higher amounts of CpG-PTO were necessary to induce the same CTL response.

CTLs are able to produce factors inducing the maturation of DCs and therefore may create a co-stimulatory milieu on their own, in particular if they are present at high frequency. The frequency of TRP-2-specific CD8⁺ T cells in blood 0 to 4 days after immunization using H-2K^{b}-dimers pulsed with TRP-2 or OVA control peptide for detection (Fig. 21) was, therefore, measured. Naive mice were vaccinated with 100 µg TRP-2 plus 5 nmol CpG-PTO both in saline, 10 µg liposomal TRP-2 plus 5 nmol CpG-PTO in saline, 10 µg TRP-2 plus 1.3 nmol CpG-PTO both liposomal, or left untreated. The frequencies of specific T cells were measured immediately after vaccination (day 0), or on day 1, 2 and 4 post-vaccination. Neither the vaccine in saline nor liposomal TRP-2 plus CpG in saline was able to induce significant numbers of specific CTLs. Only vaccination with TRP-2 and CpG encapsulated into liposomes resulted in a sizeable population of specific T cells (5% TRP-2 specific CD8⁺ T cells in the total CD8⁺ population).

Finally, the avidity of TRP-2-specific CTLs in animals immunized with liposomal or free TRP-2, respectively (Fig. 21) was compared. After multiple immunizations the frequency of IFNγ⁺ producing CD8⁺ T cells increased within splenocytes and decreased in LN (Fig. 22A). This may reflect the fact, that activated T cells leave the LN and enter the blood vessels and the spleen. Interestingly, after a single immunization with liposomal TRP-2 significantly more IFNγ⁺ T cells were detected in LN compared to mice immunized with free TRP-2 (Fig. 22B). In parallel to the IFNγ secretion assay nylon-wool column enriched splenocytes were cultured with high (10⁻⁶ mol/l) and low dose TRP-2 (10⁻⁹ mol/l), for 5 days and cell proliferation was determined using the ATPlite™-M system. A single immunization with liposomal TRP-2 generated only a few high avidity T cells as estimated by their proliferation to low dose TRP-2 (10⁻⁹ mol/l) (Fig. 22C). However, repeated immunizations raised a shift towards high avidity T cells responding only to low dose but not to high dose TRP-2. A direct comparison of liposomal vs. free TRP-2 reveals that after two immunizations the free form generates a more polyclonal response of splenocytes consisting of low and high avidity T cells. In contrast, immunization with liposomal TRP-2 generates only low numbers of low avidity T cells. To look for specificity of proliferation the H-2K^{b} binding OVA-peptide SIINFEKL was used, which induced partial proliferation at high avidity T cells due to cross priming. Splenocytes of untreated animals was used as controls demonstrating proliferation at background level.

These experiments demonstrate that specific and high-avidity T cells are generated after immunization with AVE3-encapsulated antigenic peptide. The induction of a strong immune response requires much lower concentration of encapsulated peptide and adjuvant compared to unencapsulated compounds.

### Example 12: Antitumor effects of AVE3-encapsulated TRP-2 peptide

Antitumor effects were analyzed in a subcutaneous tumor model of B16.F1 mouse melanoma cells using a prophylactic setting (Fig. 23). Mice (C57BL/6) were immunized twice into the hind footpads at a weekly interval. One week after the last immunization mice were inoculated subcutaneous with 2 x 10⁵ B16 tumor cells in a total volume of 200 µl HBSS. The tumor growth after immunization with high (100 µg TRP-2 + 5 nmol CpG-PTO) and low (10 µg TRP-2 + 1.3 nmol CpG-PTO) doses of vaccine in saline to those after immunization with low doses of liposomal vaccine (10 µg TRP-2 + 1.3 nmol CpG-PTO) was compared. The tumor mass in mice immunized with vaccine in saline was similar to that in untreated mice, and was approximately 14 times greater than that in mice immunized with liposomal vaccine 17 days after B16 inoculation (Fig. 23A). The survival time after B16 melanoma cell transfer (Fig. 23B) was also examined. No significant increase of the survival rate could be achieved with vaccine in saline (mean survival time: untreated 17 days; high dose saline 19 days; low dose saline 21 days). When mice were immunized with liposomal vaccine, the mean survival time significantly increased to 28 days (n = 5; p < 0.0172 versus untreated). One mouse treated with liposomal antigen and adjuvant completely rejected the tumor and stayed tumor free for more than 120 days.

In a second experiment, the vaccination against B16 tumor cells in a therapeutic setting (Fig. 24) was also examined. C57BL/6 mice (5 per group) were inoculated subcutaneous with 1 x 10⁵ B16 tumor cells in a total volume of 200 µl HBSS. Four days later, mice were immunized twice at a weekly interval. The tumor growth was monitored and mice were euthanized when tumor size reached 1.5 cm in diameter or ulcerated. The tumor growth after immunization with low doses of liposomal vaccine to those in untreated mice was compared. The tumor mass in untreated mice was approximately 23 times greater than that in mice immunized with liposomal vaccine 17 days after B16 inoculation (Fig. 24A). The survival time after B16 melanoma cell transfer (Fig. 24B) was also examined. A significant increase of the survival rate could be achieved with the liposomal vaccine (mean survival time: untreated 20 days; low liposomal dose 38 days; n = 10; p < 0.0012). Moreover, two mice completely rejected the tumor, stayed tumor free for more than 180 days and displayed signs of depigmentation (vitiligo) within 7 weeks after challenge demonstrating a TRP2-specific T-cell response as also melanocytes do express TRP-2.

The antitumor effect in the B16.F1 lung metastasis model using a prophylactic setting (Fig. 25) was analyzed next. For priming of cytotoxic T cells *in vivo* mice (10 per group) were immunized twice into the hind footpads at a weekly interval. One week after the last immunization, recipient C57BL/6 mice were inoculated with 2 x 10⁵ B16 tumor cells in a total volume of 200 µl HBSS via tail vein injection. On day 20 after tumor cell inoculation, mice were killed and the number of pulmonary metastases was determined. Twenty days after tumor challenge the mean number of lung metastasis in mice immunized with the liposomal vaccine (10 µg TRP-2 + 1.3 nmol CpG-PTO) was 7.7 ± 0.98 (mean ± SEM) whereas it reached 50.7 ± 4.33 in untreated mice (Fig. 25). The number of metastatic colonies was decreased by approximately 90% after liposomal vaccination (Fig. 25) indicating that the generated immune response suppresses the B 16 metastasis. Moreover, since mice vaccinated with liposomal adjuvant only (1.3 nmol CpG-PTO) were not protected against the tumor challenge (mean 44.75 ± 10.08), it was, therefore, possible to exclude that the antitumor effect was due to a CpG effect on its own.

In summary, the *in vivo* experiments in prophylactic as well as in therapeutic settings demonstrated that: (i) high doses of saline vaccine do not induce tumor protection (ii) low doses of our liposomal vaccine protects mice against a lethal id or i.v. tumor challenge, (iii) the observed effect is not a CpG-PTO effect.

### Example 13: AVE3-mediated of CpG phosphodiester oligonucleotides as adjuvant in vaccination

As shown in example 10, AVE3 are able to protect sensitive compounds such as phosphodiester oligonucleotides from degradation by plasma components. Liposomal CpG-PDO ODNs but not unencapsulated CpG-PDO ODNs were able to induce an immune response in mice as determined by the production of IFNγ-producing CD8+ cells (Fig. 26). In order to determine the optimal concentration of liposomal CpG-PDO leading to a high immunostimulation, a dose response analysis and measured the IFNγ secretion in activated T lymphocytes after vaccination with 10 µg per animal of AVE3-encapsulated TRP-2 and varying amounts of CpG-PDO was performed. 0.3 to 1.3 nmol liposomal CpG-PDO ODNs per animal induce a similar immune response (Fig. 27). Furthermore the efficacy of phosphodiester and phosphorothioate CpG-ODNs encapsulated into liposomes was compared. For this purpose a dose response analysis was performed measuring the IFNγ secretion of activated T lymphocytes. 0.3 to 1.3 nmol liposomal CpG-PDO ODNs per animal induced a higher immune response than liposomal CpG-PTO (Fig. 28). Since 0.3 nmol liposomal CpG-PTO did not lead to the production of IFNγ-producing CD8+ cells these data indicate that CpG-PDO ODNs are a more potent adjuvant than CpG-PTO at low concentrations when encapsulated in AVE3.

Then the antitumor effects in a subcutaneous tumor model of B16.F1 mouse melanoma cells using a prophylactic setting (Fig. 29) were analyzed. Mice (C57BL/6) were immunized twice into the hind footpads at a weekly interval. One week after the last immunization mice were inoculated subcutaneously with 2 x 10⁵ B16 tumor cells in a total volume of 200 µl HBSS. The tumor growth after immunization with low doses liposomal vaccine containing 0.3 nmol liposome-encapsulated phosphodiester CpG ODNs in combination with 10 µg liposomal TRP-2 per animal was analyzed. The tumor mass in untreated mice was approximately 3.5 times greater than that in mice immunized with liposomal vaccine 13 days after B16 inoculation. This finding indicates that encapsulated CpG-PO ODNs are able to serve as adjuvant to induce an antitumor immune response.

## Claims

1. A liposome, comprising in relation to the total molar lipid composition of the liposome:
a) between 20 mol% and 60 mol% cholesterol (CH); and
b) at least two components selected from the group consisting of between 20 mol% and 50 mol% of phosphatidylserine (PS), between 20 mol% and 50 mol% phosphatidylglycerol (PG), and between 20 mol% and 50 mol% phosphatidylethanolamine (PE);
c) at least one therapeutic agent, and/or at least one diagnostic agent.

2. The liposome of claim 1, wherein CH, PS, PG and/or PE is (are) present in relation to the total molar lipid composition of the liposome at a molar ratio of between 30 mol% and 36 mol%.

3. The liposome of claims 1 or 2, wherein the remaining lipid of the liposome is selected from the group consisting of glycerides, glycerophospholipides, glycerophosphinolipids, glycerophosphonolipids, sulfolipids, sphingolipids, phospholipids, isoprenolides, steroids, stearines, steroles and carbohydrate containing lipids.

4. The liposome of one of claims 1 to 3, wherein said remaining phospholipid is phosphatidylcholine (PC) or PE.

5. The liposome of one of claims 1 to 4, wherein the PS is selected from the group consisting of palmitoyloleoylphosphatidylserine. palmitoyllinoeoylphosphatidylserine, palmitoylarachidonoylphosphatidylserine, palmitoyldocosahexaenoylphosphatidylserine, stearoyloleoylphosphatidylserine, stearoyllinoleoylphosphatidylserine, stearoylarachidonoylphosphatidylserine, stearoyldocosahexaenoylphosphatidylserine, dicaprylphosphatidylserine, dilauroylphosphatidylserine, dimyristoylphosphatidylserine, diphytanoylphosphatidylserine, diheptadecanoylphosphatidylserine, dioleoylphosphatidylserine, dipalmitoylphosphatidylserine, distearoylphosphatidylserine, dilinoleoylphosphatidylserine dierucoylphosphatidylserine, didocosahexaenoyl-phospahtidylserine, PS from brain, and PS from soy bean; the PG is selected from the group consisting of palmitoyloleoylphosphatidylglycerol, palmitoyllinoleoylphosphatidylglycerol, palmitoylarachidonoylphosphatidylglycerol, palmitoyldocosahexaenoylphosphatidylglycerol, stearoyloleoylphosphatidylglycerol, stearoyllinoleoylphosphatidylglycerol, stearoylarachidonoylphosphatidylglycerol, stearoyldocosahexaenoylphosphatidylglycerol, dicaprylphosphatidylglycerol dilauroylphosphatidylglycerol, diheptadecanoylphosphatidylglycerol, diphytanoyl-phosphatidylglycerol, dimyristoylphosphatidylglycerol, dipalmitoylphosphatidylglycerol, dielaidoylphosphatidylglycerol, distearoylphosphatidylglycerol, dioleoylphosphatidylglycerol, dilinoeoylphosphatidylglycerol, diarachidonoylphosphatidylglycerol, docosahexaenoylphosphatidylglycerol, and PG from egg; and/or the PE is selected from the group consisting of palmitoyloleoylphosphatidylethanolamine, palmitoyllinoleoylphosphatidylethanolamine, palmitoylarachidonoylphosphatidylethanolamine, palmitoyldocosahexaenoylphosphatidylethanolamine, stearoyloleoylphosphatidylethanolamine, stearoyllinoleoylphosphatidylethanolamine, stearoylarachidonoylphosphatidylethanolamine, stearoyldocosahexaenoylphosphatidylethanolamine, dilauroylphosphatidylethanolamine, dimyristoylphosphatidylethanolamine, diphytanoylphosphatidylethanolamine, dipalmitoylphosphatidylethanolamine, diheptadecanoylphosphatidylethanolamine, distearoylphosphatidylethanolamine, dielaidoylphosphatidylethanolamine, dioleoylphosphatidylglycerol, dilinoeoylphosphatidylglycerol, diarachidonoylphosphatidyl-ethanolamine, docosahexaenoylphosphatidylethanolamine, PE from bacteria, PE from heart, PE from brain, PE from liver, PE from egg, and PE from soybean.

6. The liposome of one of claims 1 to 5, wherein the lipids of the lipid composition essentially consist of CH, PS, and PG; CH, PS and PE; CH, PG, and PE; or CH, PG, PS, and PE.

7. The liposome of one of claims 1 to 6, wherein the therapeutic agent is selected from the group consisting of a drug, an adjuvant or an antigen.

8. The liposome of one of claims 1 to 7, comprising at least one adjuvant and at least one antigen.

9. The liposome of claims 7 or 8, wherein the antigen is a tumor antigen, a viral antigen, a fungal antigen, a bacterial antigen an autoimmune antigen or an allergen.

10. The liposome of claim 9, wherein the tumor antigen is selected from the group consisting of T-cell-defined cancer-associated antigens belonging to unique gene products of mutated or recombined cellular genes, in particular cyclin-dependent kinase 4 (CDK4), p15^{Ink4b}, p53, AFP, β-catenin, caspase 8, p53, p21^{Ras} mutations, Bcr-abl fusion product, MUM-1 MUM-2, MUM-3, ELF2M, HSP70-2M, HST-2, KIAA0205, RAGE, myosin/m, 707-AP, CDC27/m, ETV6/AML, TEL/Aml1, Dekcain, LDLR/FUT, Pml-RARα, TEL/AMLI; Cancer-testis (CT) antigens, in particular NY-ESO-1, members of the MAGE-family (MAGE-A1, MAGE-A2, MAGE-A3, MAGE-A4, MAGE-A6 MAGE-10, MAGE-12), BAGE, DAM-6, DAM-10, members of the GAGE-family (GAGE-1, GAGE-2, GAGE-3, GAGE-4, GAGE-5, GAGE-6, GAGE-7B, GAGE-8), NA-88A, CAG-3, RCC-associated antigen G250; Tumor virus antigens, in particular human papilloma virus (HPV)-derived E6 E7 oncoproteins, Epstein Barr virus EBNA2-6, LMP-1, LMP-2; overexpressed or tissue-specific differentiation antigens, in particular gp77, gp100, MART-1/Melan-A, p53, tyrosinase, tyrosinase-related protein (TRP-1 and TPR-2), PSA, PSM, MC1R; widely expressed antigens, in particular ART4, CAMEL, CEA, CypB, HER2/neu, hTERT, hTRT, iCE, Muc1, Muc2, PRAME RU1, RU2, SART-1, SART-2, SART-3, and WT1; and fragments and derivatives thereof.

11. The liposome of claim 9, wherein the viral antigen is derived from a virus selected from the group of virus consisting of Retroviridae, in particular HIV-1 and HIV-LP; Picomaviridae, in particular polio virus and hepatitis A virus; enterovirus, in particular human coxsackie virus, rhinovirus, echovirus; Calciviridae, in particular strains that cause gastroenteritis; Togaviridae, in particular equine encephalitis virus and rubella virus; Flaviridae, in particular dengue virus, encephalitis virus and yellow fever virus; Coronaviridae, in particular coronavirus; Rhabdoviridae, in particular vesicular stomatitis virus and rabies virus; Filoviridae, in particular Ebola virus or and Marburg virus; Paramyxoviridae, in particular parainfluenza virus, mumps virus, measles virus and respiratory syncytical virus; Orthomyxoviridae, in particular influenza virus; Bungaviridae, in particular Hantaan virus, bunga virus, phlebovirus and Nairo virus; Arena viridae, in particular hemorrhagic fever virus; Reoviridae, in particular reovirus, orbivirus and rotavirus; Bimaviridae; Hepadnaviridae, in particular Hepatitis B virus; Parvovirida, in particular parvovirus; Papovaviridae, in particular papilloma virus, simian virus-40 (SV40) and polyoma virus; Adenoviridae; Herpesviridae, in particular herpes simplex virus (HSV) 1 and 2, varicella zoster virus, cytomegalovirus (CMV), herpes virus; Poxviridae, in particular variola virus, vaccinia virus and pox virus; and Iridoviridae, in particular African swine fever virus; and Hepatitis C.

12. The liposome of claim 9, wherein the fungal antigen is derived from a fungus selected from the group consisting of *Cryptococcus* species, in particular *Cryptococcus neoformans, Histoplasma* species, in particular *Histoplasma capsulatum, Coccidioides* species, in particular *Coccidioides immitis, Blastomyces* species, in particular Blastomyces *dermatitidis, Chlamydia* species, in particular *Chlamydia trachomatis,* and *Candida* species, in particular *Candida albicans*.

13. The liposome of claim 9, wherein the bacterial antigen is derived from a bacterium selected from the group consisting of Helicobacter species, in particular *Helicobacter pyloris*; Borelia species, in particular *Borelia burgdorferi;* Legionella species, in particular *Legionella pneumophilia*; Mycobacteria species, in particular *M. tuberculosis, M. avium, M. intracellulare, M. kansasii, M. gordonae;* Staphylococcus species, in particular *Staphylococcus aureus*; Neisseria species, in particular *N. gonorrhoeae, N. meningitidis;* Listeria species, in particular *Listeria monocytogenes;* Streptococcus species, in particular *S. pyogenes, S. agalactiae; S. faecalis; S. bovis, S. pneumoniae*; anaerobic Streptococcus species; pathogenic Campylobacter species; Enterococcus species; Haemophilus species, in particular *Haemophilus influenzae*; Bacillus species, in particular *Bacillus anthracis*; Corynebacterium species, in particular *Corynebacterium diphtheriae*; Erysipelothrix species, in particular *Erysipelothrix rhusiopathiae*; Clostridium species, in particular *C. perfringens, C. tetani*; Enterobacter species, in particular *Enterobacter aerogenes*, Klebsiella species, in particular *Klebsiella pneumoniae*, Pasturella species, in particular *Pasturella multocida,* Bacteroides species; Fusobacterium species, in particular *Fusobacterium nucleatum;* Streptobacillus species, in particular *Streptobacillus moniliformis;* Treponema species, in particular *Treponema pertenue;* Leptospira; pathogenic Escherichia species; and Actinomyces species, in particular *Actinomyces israelli.*

14. The liposome of claim 7, wherein the drug is selected from the group consisting of analgesics; antirheumatics; anthelminthics; antiallergics; antianemics; antiarrhythmics; antibiotics; angiogenesis inhibitors; antiinfectives; antidemenics (nootropics); antidiabetics; antidotes; antiemetics; antivertiginosics; antiepileptics; antihemorrhagics; antihypertonics; antihypotonics; anticoagulants; antimycotics; antitussive agents; antiviral agents; beta-receptor and calcium channel antagonists; broncholytic and antiasthmatic agent; chemokines; cytokines, in particular immune modulatory cytokines; mitogens; cytostatics; cytotoxic agents and prodrugs thereof; dermatics; hypnotics and sedatives; immunosuppressants; immunostimulants in particular activators of NF-κB, MAP kinases, STAT proteins and/or protein kinase B/Akt; peptide or protein drugs; in particular hormones and physiological or pharmacological inhibitors of mitogens, chemokines, or cytokines or their respective prodrugs.

15. The liposome of one of claims 7 to 14, wherein the adjuvant is selected from the group consisting of unmethylated DNA, in particular unmethylated DNA comprising CpG dinucleotides (CpG motif), in particular CpG ODN with phosphorothioate (PTO) backbone (CpG PTO ODN) or phosphodiester (PO) backbone (CpG PO ODN); gel-like precipitates of aluminum hydroxide (alum); bacterial products from the outer membrane of Gram-negative bacteria, in particular monophosphoryl lipid A (MPLA), lipopolysaccharides (LPS), muramyl dipeptides and derivatives thereof; synthetic lipopeptide derivatives, in particular Pam₃Cys; lipoarabinomannan; peptidoglycan; zymosan; heat shock proteins (HSP), in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, interleukin (IL-)2, IL-6, IL-7, IL-18, type I and II, interferons, in particular interferon-gamma, TNF-alpha; oil in water emulsions, in particular MF59 consisting of squalene; Tween 80 and Span 85 (sorbitan-trioleate) and QS-21, non-ionic block polymers, in particular Poloxamer 401, saponins and derivatives thereof; polyphosphazene; N-(2-Deoxy-2-L-leucylamino-β-D-glucopyranosyl)-N-octadecyldodecanoylamide hydroacetate (BAY R1005), 1α, 25-dihydroxyvitamin D3 (calcitriol); DHEA; murametide [MDP(Gln)-OMe]; murapalmitine; polymers of lactic and/or glycolic acid; polymethyl methacrylate; sorbitan trioleate; squalane; stearyl tyrosine; squalene; theramide, synthetic oligopeptides, in particular peptides presented by MHC-class II.

16. The liposome of one of claims 1 to 15, wherein the diagnostic agent is selected from the group consisting of an electron dense molecule, a paramagnetic molecule, a superparamagnetic molecule, a radioactive molecule, a non-radioactive isotope, and a fluorescent molecule.

17. The liposome of one of claims 1 to 16, wherein the ratio of the molar amount of therapeutic agent or diagnostic agent to the molar amount of total lipids is between 1:100 and 1:10, preferably between 1:50 and 1:20.

18. The liposome of one of claims 1 to 17, wherein the liposome has a diameter of between 50 and 200 nm, preferably between 80 and 150 nm.

19. The liposome of one of claims 1 to 18, wherein the liposome has a negative surface charge.

20. The liposome of one of claims 1 to 19, wherein a targeting moiety is attached to the liposome.

21. The liposome of one of claims 1 to 20, which is dried, preferably freeze dried.

22. A method for producing the liposome of one of claims 1 to 21, comprising the steps of:
a) forming a suspension of lipids comprising CH, and at least two components selected from the group consisting of PS, PG, and PE one or more therapeutic and/or diagnostic agent and a liquid medium and
b) homogenizing the suspension.

23. The method of claim 22, wherein the therapeutic and/or the diagnostic agent and/or the lipids is (are) essentially not soluble in the liquid medium.

24. The method of claim 22 or 23, wherein the liquid medium is selected from the group consisting of H₂0, aqueous salt solution, and buffer solution.

25. A liposome produced by the method of one of claims 22 to 24.

26. A liposomal composition comprising the liposome of claims 1 to 21 or claim 25 and has at least one further component selected from the group consisting of an adjuvant, additive, buffer and auxiliary substance.

27. The liposomal composition of claim 26, wherein the adjuvant is selected from the group of adjuvants consisting of unmethylated DNA, in particular unmethylated DNA comprising CpG dinucleotides, in particular CpG PTO ODN or CpG PO ODN; alum; bacterial products from the outer membrane of Gram-negative bacteria, in particular MPLA, lipopolysaccharides (LPS), muramyl dipeptides and derivatives thereof; synthetic lipopeptide derivatives, in particular Pam₃Cys; lipoarabinomannan; peptidoglycan; zymosan; HSP, in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, IL-2, IL-6, IL-7, IL-18, type I and II, interferons, in particular interferon-gamma, TNF-alpha; oil in water emulsions, in particular MF59 consisting of squalene; Tween 80 and Span 85 and QS-21, non-ionic block polymers, in particular Poloxamer 401, saponins and derivatives thereof; polyphosphazene; BAY R1005, calcitriol; DHEA; murametide [MDP(Gln)-OMe]; murapalmitine; polymers of lactic and/or glycolic acid; polymethyl methacrylate; sorbitan trioleate; squalane; stearyl tyrosine; squalene; theramide, synthetic oligopeptides, in particular peptides presented by MHC-class II.

28. Use of a liposome of one of claims 1 to 21 or claim 25 or the liposomal composition of claim 31 or 32 for the production of a medicament for the prevention or therapy of proliferative diseases, infectious diseases, vascular diseases, rheumatoid diseases, inflammatory diseases, immune diseases, and allergies.

29. The use of claim 28, wherein the proliferative disease is selected from the group consisting of carcinomas of the gastrointestinal or colorectal tract, liver, pancreas, kidney, bladder, prostate, endometrium, ovary, testes, melanoma, dysplastic oral mucosa, invasive oral cancers, small cell and non-small cell lung carcinomas, hormone-dependent breast cancers, hormone independent breast cancers, transitional and squamous cell cancers, neurological malignancies including neuroblastoma, gliomas, astrocytomas, osteosarcomas, soft tissue sarcomas, hemangioamas, endocrinological tumors, hematologic neoplasias including leukemias, lymphomas, and other myeloproliferative and lymphoproliferative diseases, carcinomas in situ, hyperplastic lesions, adenomas, fibromas, histiocytosis, chronic inflammatory proliferative diseases, vascular proliferative diseases and virus-induced proliferative diseases.

30. The use of claims 28 or 29, wherein an adjuvant and/or a cytokine is (are) administered prior, simultaneously or after administration of the liposome or liposomal composition.

31. The use of claim 30, wherein the adjuvant is selected from the group of adjuvants consisting of unmethylated DNA, in particular unmethylated DNA, in particular unmethylated DNA comprising CpG dinucleotides, in particular CpG PTO ODN or CpG PO ODN; alum; bacterial products from the outer membrane of Gram-negative bacteria, in particular MPLA, lipopolysaccharides (LPS), muramyl dipeptides and derivatives thereof; synthetic lipopeptide derivatives, in particular Pam₃Cys; lipoarabinomannan; peptidoglycan; zymosan; HSP, in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, IL-2, IL-6, IL-7, IL-18, type I and II, interferons, in particular interferon-gamma, TNF-alpha; oil in water emulsions, in particular MF59 consisting of squalene; Tween 80 and Span 85 and QS-21, non-ionic block polymers, in particular Poloxamer 401, saponins and derivatives thereof; polyphosphazene; BAY R1005, calcitriol; DHEA; murametide [MDP(Gln)-OMe]; murapalmitine; polymers of lactic and/or glycolic acid; polymethyl methacrylate; sorbitan trioleate; squalane; stearyl tyrosine; squalene; theramide, synthetic oligopeptides, in particular peptides presented by MHC-class II.
